# EUROPEAN PATENT APPLICATION

(11) **EP 4 484 547 A1**
(43) Date of publication of application: **01.01.2025**
(21) Application number: 23382666.8
(22) Date of filing: 29.06.2023
(51) Int. Cl.: C12N 1/20, A01N 63/20

(54) **PARARHIZOBIUM STRAINS, COMPOSITIONS AND USES THEREOF AS PLANT GROWTH STIMULANTS**

(71) Applicant: Universitat de Barcelona, 08028 Barcelona (ES)
(72) Inventor: ALCÁZAR HERNÁNDEZ, Rubén, 08028 Barcelona (ES); ATANASOV, Kostadin Evgeniev, 08028 Barcelona (ES)
(74) Representative: Hoffmann Eitle

(57) **Abstract**

The present invention provides a *Pararhizobium* strain selected from: (a) a strain deposited in the Spanish Type Culture Collection (CECT) with the accession number CECT30825; (b) a strain deposited in the Spanish Type Culture Collection (CECT) with the accession number CECT30826; or (c) a mutant thereof, wherein the mutant: (i) is obtained from CECT30825 or CECT30826 strain, and (ii) retains one or more of the following properties: (ii.1) induction of plant tolerance to chilling temperature; (ii.2) induction of plant tolerance to freezing temperature; (ii.3) induction of plant growth; and (ii.4) increased plant yield.

The invention further provides compositions, uses and methods based on the strains of the invention.

Advantageously the strains of the invention are of broad-spectrum, providing an effective protective effect in several abiotic stresses, such as low temperatures and saline environment.

## Description

### FIELD OF THE INVENTION

The present invention relates to the field of plant stimulants. In particular, the present invention provides new strains of *Pararhizobium* genera, as well as compositions and uses thereof in promoting plant growth.

### STATE OF THE ART

Controlling plant growth and mitigating the adverse impact of environmental stressors are crucial factors that dictate the yield of cultivated crops. Abiotic stresses can be prevented by optimizing plant growth conditions through the use of plant biostimulants. Plant biostimulants include a diversity of substances and microorganisms that enhance plant growth by improving plant metabolism to induce yield increases and enhanced crop quality; increase plant stress tolerance and recovery from stresses; facilitate nutrient assimilation, translocation and use; and their effects operate through different mechanisms than fertilizers (Calvo *et al.,* 2014). Over the past few decades, there has been a significant rise in the application of microbial inoculants in agriculture (Li *et al.,* 2022). Microbial inoculants mainly include free-living bacteria, fungi, and arbuscular mycorrhizal fungi isolated from different sources (Berg, 2009).

Plants face various environmental stresses, such as salt stress, extreme temperatures, drought, and flooding, among others, which can adversely affect their growth and productivity. The use of beneficial microbes offers a promising solution to alleviate these stresses and improve plant performance. Salt stress is a major threat to crop productivity, and beneficial microbes have been shown to enhance plant salt tolerance (Kumawat *et* al., 2023). For example, pea plants inoculated with *Variovorax paradoxus* 5C-2, which produces 1-aminocyclopropane-1-carboxylate (ACC) deaminase, showed increased photosynthetic rate, electron transport, balanced ion homeostasis, and biomass under salt stress at 70 mM and 130 mM NaCl (Q., Wang *et al.,* 2016). Okra *(Abelmoschus esculentus* L.) plants treated with plant growth promoting rhizobacteria (PGPR) producing ACC also showed enhanced salt tolerance, increased antioxidant enzyme activities, and upregulated ROS pathway genes (Habib *et al.,* 2016). Maize seedlings inoculated with *Bacillus amyloliquefaciens* SQR9 showed enhanced salt stress tolerance, including improved chlorophyll content, peroxidase/catalase activity, glutathione content for scavenging ROS, and reduced sodium levels in the plant (Chen *et al.,* 2016). Wheat plants inoculated with the halotolerant *Dietzia natronolimnaea* showed upregulation of genes involved in the ABA-signaling cascade, salt overly sensitive (SOS) pathway, ion transporters, and antioxidant enzymes (Bharti *et al.,* 2016). In tomato, treatment with strains of *Bacillus sp.* led to higher levels of total soluble sugar, proline, and chlorophyll as well as higher antioxidant enzyme activities in salt-stressed plants (Patani *et al.,* 2023).

Extreme temperatures, such as cold and heat stress, also pose significant challenges to crop productivity. The primary factors that cause low-temperature stress are freezing (temperatures below 0 °C) and chilling (temperatures between 0-15 °C). When plants experience freezing stress, the formation of ice crystals in the cells results in freezing dehydration and electrolyte leakage (Mitra *et al.,* 2021). Some beneficial microbes have been shown to improve plant performance under extreme temperature conditions. For example, inoculation with *Serratia nematodiphila,* a gibberellin-producing PGPR, increased pepper growth under low temperature stress conditions (Kang *et al.,* 2015). *Burkholderia phytofirmans* PsJN modulated sugar metabolism and reduced chilling damage to grapevine plantlets exposed to low temperature stress (Fernandez *et al.,* 2012) and reduced the impact of freezing temperatures on photosynthesis in *Arabidopsis thaliana* (Su *et al.,* 2015). Inoculation of tomato plants exposed to low temperatures with *Pseudomonas vancouverensis* OB155 and P. *frederiksbergensis* OS261 increased the expression of cold-acclimation genes and the antioxidant activity in leaf tissues (Subramanian and Smith, 2015). A consortium of three PGPR strains *Bacillus cereus* AR156, *B. subtilis* SM21, and *Serratia* sp. XY21 provided enhanced chilling (4 °C) tolerance in tomato seedlings by stimulation of faster and higher accumulations of MDA, H₂O₂, soluble sugar and proline accumulation (C., Wang *et al.,* 2016). In rice, a consortium of *Bacillus amyloliquefaciens* Bk7 and *Brevibacillus laterosporus* B4 enhanced plant growth and reduced stress symptoms in rice seedlings subjected to 0 ± 5 °C for 24 h, in correlation with reduced MDA and electrolyte leakage, increased proline, cholorophyll content and antioxidant enzyme activities (Kakar *et al.,* 2016).

In spite of the efforts made there is still the need of providing plant growth promoters.

### SUMMARY OF THE INVENTION

The present inventors have identified new strains of the genera Pararhizobium with the ability of promoting plant growth under different abiotic stress conditions, such as low temperatures (below 0 °C) and high salinity.

As it is shown below, the strains, applied on aerial parts of the plant or in the soil, provided an efficient protective effect under the adverse conditions of temperature and salinity. Not only that, but also, they induced an increase in weight and number of seeds, providing, therefore, an increase in the yield and biomass.

In view of the data provided below, therefore, the strains of the invention mean a great advance in the field of plant stimulants because they are of broad-spectrum, providing both protective and stimulant effects under different abiotic stress conditions.

Thus, in a first aspect the present invention provides a *Pararhizobium* strain selected from:
(a) a strain deposited in the Spanish Type Culture Collection (CECT) with the accession number CECT30825,
(b) a strain deposited in the Spanish Type Culture Collection (CECT) with the accession number CECT30826, or
(c) a mutant thereof, wherein the mutant: (i) is obtained from CECT30825 or CECT30826 strain, and (ii) retains one or more of the properties of inducing: (ii.1) tolerance to chilling or freezing temperature, (ii.2) tolerance to saline stress; (ii.3) plant growth, or (ii.4) increased yield.

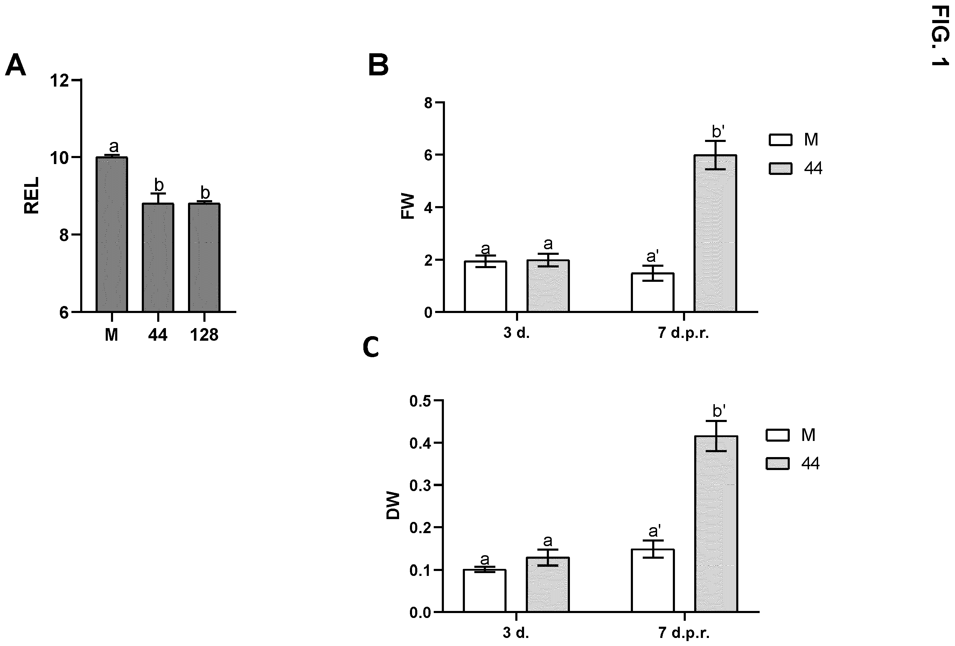

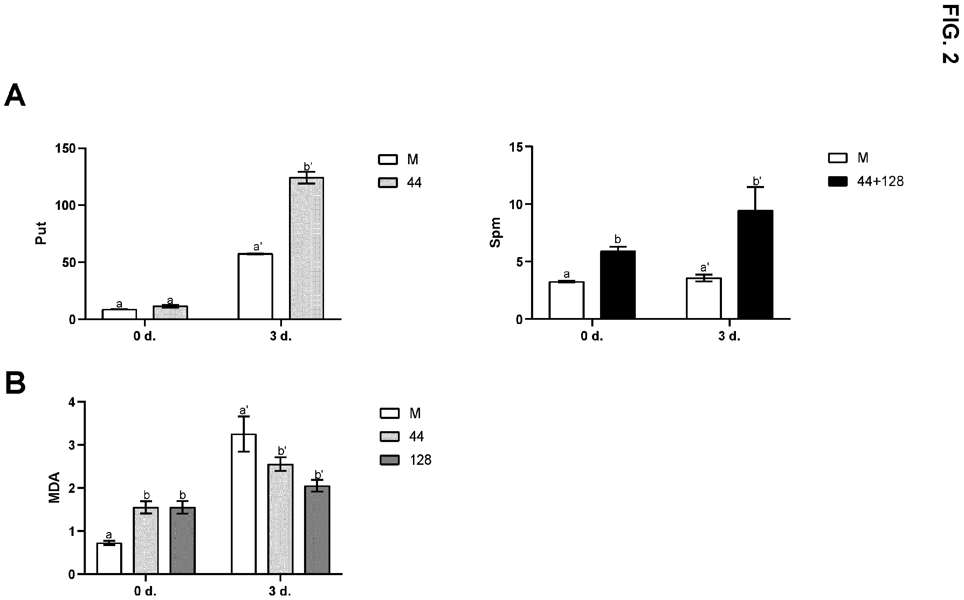

As it is shown below, the strains of the invention protected against low temperatures, significantly reducing the electrolyte leakage (Fig. 1A). Without being bound to the theory, the present inventors believe that this can be due, at least in part, to the induction of a higher production of protective polyamines, such as putrescine or spermine (Fig. 2A), as well as to a reduction in the oxidation processes related to stress such as lipid peroxidation (as it is shown in Fig 2B, MDA levels).

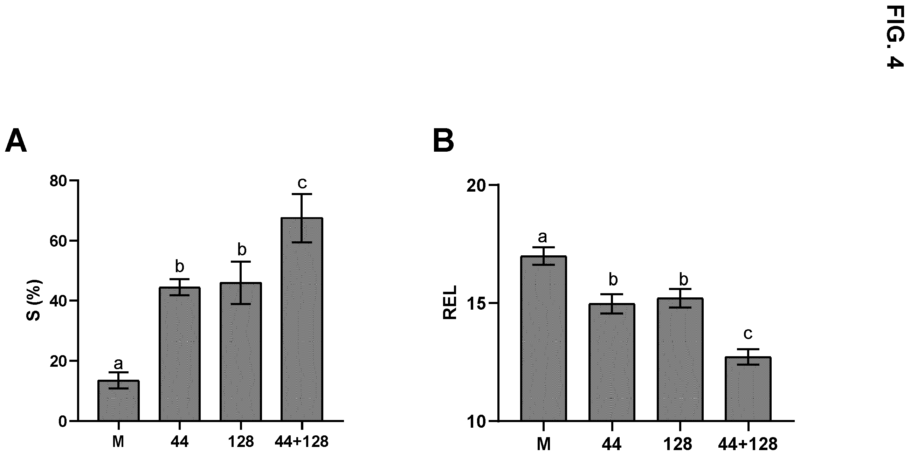

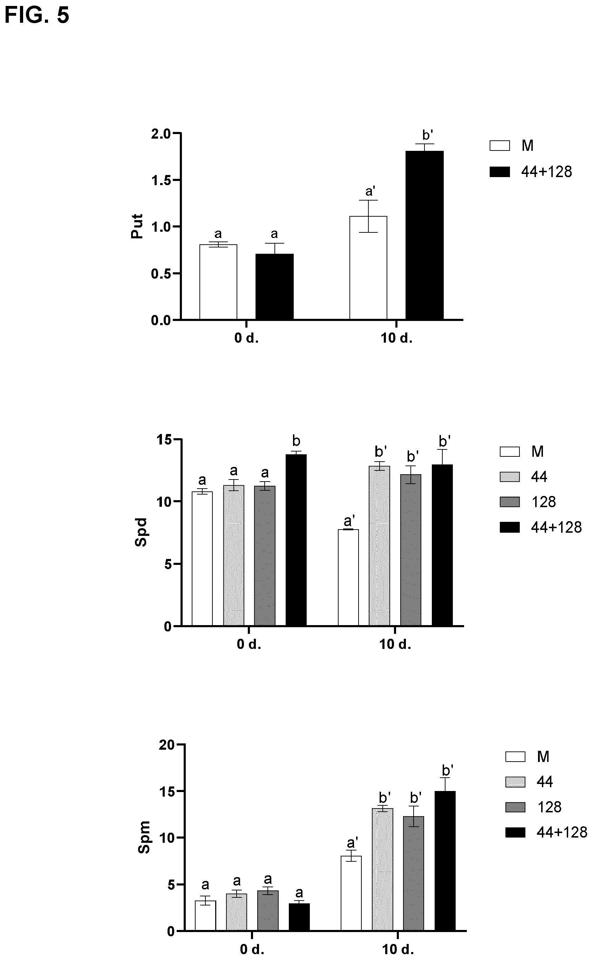

The same advantageous protective effect was also found under high saline conditions. Fig. 4 shows that the strains of the invention provided a significant protective effect, again related to the induction of an increased production of protective polyamines such as putrescine, spermine or spermidine (see Fig. 5).

Not only that, but also, a significant increase in the biomass was found just 7 days after-recovering (see Fig. 1B and 1C), in the weight of the fruits (Fig. 6A) and in seed production (Fig. 7A and B). Advantageously, the organoleptic properties of the fruits, using the strains of the invention, were improved by decreasing the titratable acidity (TA) and increasing the total soluble solid TSS/TA ratio (Fig. 7C to E).

In the present invention the terms "strain 44" and "CECT30825", referring to the strain of the invention, are used interchangeably.

In the present invention the terms "strain 128" and "CECT30826", referring to the strain of the invention, are used interchangeably.

The strains of the invention were isolated from an agricultural soil collected in Collserola, Barcelona (Spain) and deposited, according to the Budapest Treaty, at the Colección Española de Cultivos Tipo (CECT), Universidad de Valencia, C.P 46980 Catedrático Agustin Escardino N° 9, Paterna, Valencia (Spain), on April 25, 2023. They were, in addition, declared viable.

In a second aspect the present invention provides a bacterial culture comprising the strain or the mutant thereof according to the first aspect of the invention.

In a third aspect the present invention provides a supernatant derived from the strain as defined in the first aspect of the invention, said supernatant being obtainable by a process comprising: (i) inoculating the strain in a suitable culture medium; (ii) subjecting the inoculated culture medium to suitable growth conditions; (iii) separating the cells from the culture medium of step (ii); (iv) collecting the supernatant; and (v) optionally subjecting the supernatant to a concentration step.

In a fourth aspect the present invention provides a process for obtaining a viable cell suspension derived from the strain as defined in the first aspect of the invention, the process comprising: (i) inoculating the strain in a culture medium, (ii) subjecting the inoculated culture medium of the step (i) to conditions suitable for growth of the strain, and (iii) optionally subjecting the medium resulting from step (ii) to a concentration step.

In a fifth aspect the present invention provides a method to obtain a mutant of the strain of CECT30825, as defined in claim 1, comprising the step of subjecting the strain CECT30825 or CECT30826 to a DNA recombinant technique, preferably mutagenesis.

In a sixth aspect the present invention provides a composition comprising the strain according to claim 1 or the bacterial culture according to any one of the claims 2-3 or the supernatant according to claim 4, and one or more agriculturally acceptable compounds.

In a seventh aspect the present invention provides a kit comprising an effective amount of the strain as defined in the first aspect of the invention, the bacterial culture according to the second aspect of the invention, the supernatant according to the third aspect of the invention, or the composition according to the fourth aspect of the invention.

In an eighth aspect the present invention provides the use of the *Pararhizobium* strain as defined in the first aspect of the invention, the bacterial culture as defined in the second aspect of the invention, the supernatant as defined in the third aspect of the invention, or the composition as defined in the sixth aspect of the invention, as a growth plant promoter.

In a ninth aspect, the present invention provides the use of the *Pararhizobium* strain as defined in the first aspect of the invention, the bacterial culture as defined in the second aspect of the invention, the supernatant as defined in the third aspect of the invention, or the composition as defined in the sixth aspect of the invention, for increasing abiotic stress tolerance, biomass and/or yield of a plant.

In a tenth aspect the present invention provides a method for promoting the growth in a plant, the method comprising applying the strain to the plant as defined in the first aspect of the invention, the bacterial culture as defined in the second aspect of the invention, the supernatant as defined in the third aspect of the invention, or the composition as defined in the sixth aspect of the invention.

In an eleventh aspect the present invention provides a method for increasing abiotic stress tolerance, biomass and/or yield of a plant, the method comprising applying to the plant the strain as defined in the first aspect of the invention, the bacterial culture as defined in the second aspect of the invention, the supernatant as defined in the third aspect of the invention, or the composition as defined in the sixth aspect of the invention.

### BRIEF DESCRIPTION OF DRAWINGS

**Fig. 1. (A)** Relative electrolyte leakage (REL) expressed as % and (B) fresh weight (FW) and dry weight (DW) (g), determined in tomato plants inoculated with *Pararhizobium* strains 44, 128 or mock (M, water) after three days (3 d.) of exposure to cold (4 °C) conditions. (C) Fresh and dry weight at 7-day post-recovery (7 d.p.r) from cold treatment.
The values represent the mean ± standard deviation (S.D.) from four biological replicates per treatment. Different letters indicate significant differences (p<0.05) according to two-way ANOVA followed by Tukey's post-hoc test.
Fig. 2. Determination of (A) free polyamines Putrescine (Put) and Spermine (Spm) (nmol/g fresh weight) (B) malondialdehyde (MDA) content (nmol/mg fresh weight) before (0 days, 0 d.) and after 3 days (3 d.) of cold stress (4 °C) in tomato (var. Moneymaker) plants previously inoculated with *Pararhizobium* strains 44 , 128, combined 44+128 or mock (M, water). The values represent the mean ± standard deviation (S.D.) from four biological replicates per treatment. Different letters indicate significant differences (p<0.05) according to two-way ANOVA followed by Tukey's post-hoc test.

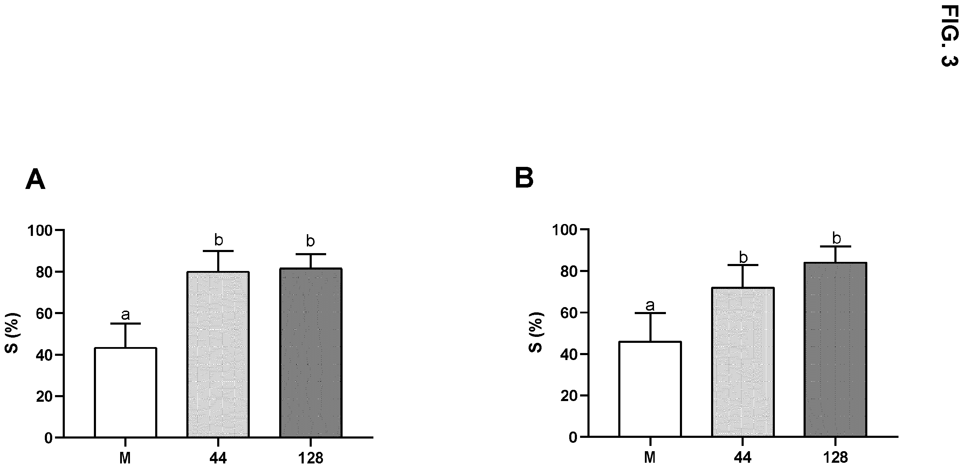

**Fig. 3**. Effect of **(A)** spray inoculation and **(B)** soil inoculation with *Pararhizobium* strains 44, 128 or mock (M, water) on freezing (- 4 °C) tolerance, measured as survival rates (S (%)) in rapeseed plants. The values represent the mean ± standard deviation (S.D.) from ten biological replicates per treatment. Different letters indicate significant differences (p<0.05) according to two-way ANOVA followed by Tukey's post-hoc test.
**Fig. 4. (A)** Determination of survival rates (S (%)) in rapeseed seedlings germinated and grown on half-strength Murashige and Skoog's (MS) media supplemented with 300 mM NaCl in the presence of 10⁸ CFU/ml of *Pararhizobium* strains 44, 128, the combination of both (44 + 128) or mock (M, no bacteria). Seedlings were exposed to saline conditions for three weeks. Survival rates were determined by the number of plants that resumed growth after a 7-day recovery in half-strength MS media without salt. **(B)** Relative electrolyte leakage (REL) expressed as % of rapeseed plants spray-inoculated with *Pararhizobium* strains 44, 128, the combination of both (44 + 128) or mock (M, water) after 10 days of irrigation with 250 mM NaCl. REL was determined on day 28 after germination. The values represent the mean ± standard deviation (S.D.) from ten biological replicates per treatment. Different letters indicate significant differences (p<0.05) according to two-way ANOVA followed by Tukey's post-hoc test.
**Fig. 5****.** Determination of free polyamines Putrescine (Put), Spermidine (Spd) and Spermine (Spm) (nmol/g fresh weight) before (0 days, 0 d.)) and after 10 days (10 d.) of salt stress (250 mM NaCl) treatment in rapeseed plants previously inoculated with *Pararhizobium* strains 44, 128, combined 44+128 or mock (M, water). The values represent the mean ± standard deviation (S.D.) from four biological replicates per treatment. Different letters indicate significant differences (p<0.05) according to two-way ANOVA followed by Tukey's post-hoc test.

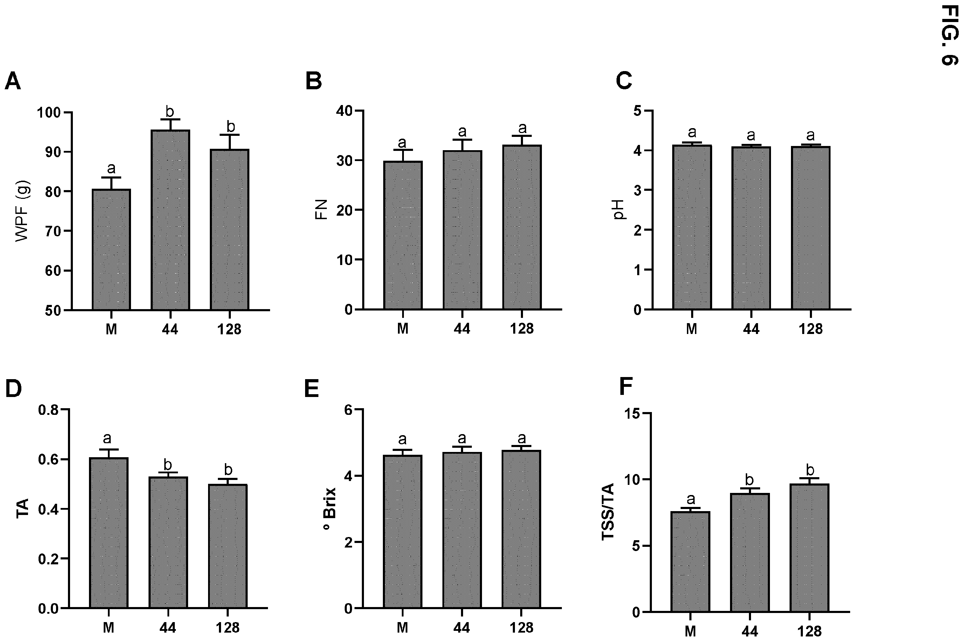

**Fig. 6****.** Tomato (var. Moneymaker) plants were germinated and grown on soil under greenhouse conditions and spray-inoculated with 10⁸ CFU/ml of *Pararhizobium* strains 44, 128 or mock (M, water) on day 14, 35 and 63 after germination. Fruits were harvested 120 days after germination at red ripening stage, and different parameters determined: **(A)** weight per fruit (WPF) (g), **(B)** number of fruits per plant (FN), **(C)** pH, **(D)** titratable acidity (TA), **(E)** total soluble solids (TSS or °Brix), and **(F)** TSS/TA ratio. The values represent the mean ± standard deviation (S.D.) from at least 12 biological replicates per treatment. Different letters indicate significant differences (p<0.05) according to two-way ANOVA followed by Tukey's post-hoc test.

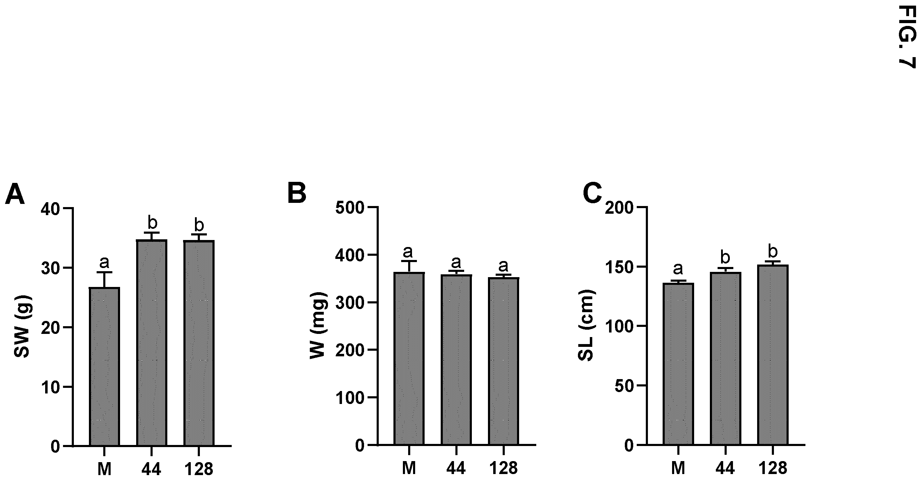

**Fig. 7****.** Rapeseed plants were germinated and grown on 10-liter pots using agricultural soils without fertilization. Plants were spray-inoculated with 10⁸ CFU/ml of *Pararhizobium* strains 44, 128 or mock (M, water) on day 14, 35 and 63 after germination. Seeds were harvested after ripening for the determination of **(A)** seed weight per plant (SW) (g) and **(B)** weight per 100 seeds (W) (mg). **(C)** Stem length (SL) (cm) was determined at ripening stage. The values represent the mean ± standard deviation (S.D.) from at least 24 biological replicates per treatment. Different letters indicate significant differences (p<0.05) according to two-way ANOVA followed by Tukey's post-hoc test.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings.

The invention provides strains CECT30825, CECT30286, and any mutant thereof.

The term "mutant" is also understood, in the context of the invention, as a "variant" of the strains CECT30825 and CECT30826.

By using the deposited strain as starting material, the skilled person in the art can routinely, by genetic engineering techniques such as mutagenesis or bacterial recombination techniques, obtain mutants that maintain the herein described relevant features and advantages of the strain of the invention. In an embodiment of the invention, the mutant is a genetically modified mutant obtained by random mutagenesis (i.e. using chemical or physical agents) or by site-directed mutagenesis, combinatorial mutagenesis or insertional mutagenesis. In another embodiment of the first aspect of the present invention, the mutant is obtained by using recombinant technology, for example using transformation (for example, by electroporation, heat-shock or using divalent cation solutions, such as calcium chloride), transduction or conjugation techniques. By using recombinant technology, a plasmid can be included in the bacterial strain, said plasmid can comprise antibiotic resistant genes or genes that serve for the selection of the mutant. Examples of genetic engineering techniques can be found in (Sambrook and Russell, 2001).

As mentioned above, the mutant provided by the present invention has to maintain the effect shown by the strains of the invention, which are further illustrated below.

In order to determine whether the mutant maintains the ability to induce growth and tolerance to abiotic stress, several well-known protocols can be followed. The protocols that are included below, in the examples, are illustrative and non-limitative examples.

In a second aspect the present invention provides a bacterial culture.

In one embodiment of the second aspect the bacterial culture is an inoculation product.

By "inoculation product" it is understood a product obtained after inoculating the strain in a suitable culture medium, subjecting the inoculated culture medium to suitable growth conditions.

The strain of the invention may be inoculated in the culture medium at a final concentration comprised from 3 to 7% v/v, for example from 5 to 7% v/v, for example of 5% v/v. Preferably the inoculated culture is in an exponential growth phase. Suitable culture media for the growth of the strain of the invention are synthetic media, such as LB (lysogenic broth) and PM (saline production medium), or media of plant origin such as molasses (e.g. from sugar cane, beets and others). Suitable conditions for strain's growth are temperatures comprised from 4 to 35 °C, pH comprised 4 to 10 (for example 7 to 10), and oxygen concentration comprised from 10 to 100%. In a particular embodiment, the conditions for the growth of the strain are temperatures of 28-30 °C, pH of 7-8, and oxygen concentration of 50%. During the growth of the strain of the invention stirring can be used.

In another embodiment of the second aspect of the invention, optionally in combination with any of the embodiments provided above or below, the bacterial culture comprises the strain of the invention inactivated.

The term "inactivated" means that the micro-organism is not able to form colonies. In one embodiment, the inactivated micro-organisms have the cell membrane intact or broken.

In another embodiment of the second aspect of the invention, optionally in combination with any of the embodiments provided above or below, the inoculation product comprises the strain of the invention inactivated. By "inoculation product comprising the strain of the invention inactivated" refers to a product obtained after inoculating the strain in a suitable culture medium, subjecting the inoculated culture medium to suitable growth conditions, and then inactivating the strain.

A third aspect of the invention refers to a supernatant from the strains of the invention.

In a particular embodiment of the sixth aspect of the invention, optionally in combination with any of the embodiments provided above or below, the supernatant is from the strain CECT30825.

The term "derived from the strain" means that the suspension is obtained from the strain as defined in the first or ninth aspect of the present invention.

In the context of the invention, the processes for obtaining the supernatant and the viable cell suspension, comprises the steps of inoculation and induction of cell growing, which can be performed under conditions which can be easily determined by the skilled person in the art.

In another embodiment of the process to obtain the supernatant or the viable cell suspension, the cells, after completing the growth step, are separated from the medium to obtain a concentrated suspension. Suitable separation techniques include centrifugation or filtration of the culture. Carrying out the centrifugation of the culture, for example, at a minimum of 5000 rpm, cells are obtained in the pellet, which are resuspended in part of the culture medium or in a suitable buffered medium such that the strain concentration is approximately about 1×10⁵- 1×10¹² CFU/mL (10⁵ CFU /mL, 10⁶ CFU /mL, 10⁷ CFU /mL, 10⁸ CFU /mL, 10⁹ CFU /mL, 10¹⁰ CFU /mL, 10¹¹ CFU /mL or 10¹² CFU /ml).

In one embodiment of the process of the fourth aspect of the invention, once the suspension is obtained, it may be subjected to a dehydration step. Dehydration can be carried out through a lyophilisation process. Alternatively, the suspension can be dehydrated by fluidised bed drying. Another option is to dehydrate the suspension by spray drying or drying in an oven under vacuum. In this regard, another advantageous feature of the strain of the invention is that it exhibits high resistance to dehydrating processes, which are routine in obtaining microorganisms on an industrial scale. In order to improve cell viability, an inert osmotic protector ingredient can be added to the suspension before carrying out the dehydration process.

In another particular embodiment of the process of the fourth aspect of the invention, the process comprises re-suspending the cells resulting from the separation step in a suitable buffer to yield a cell concentrated suspension.

The cells obtained with the process of the fourth aspect of the invention may then be used directly, resuspended to a desired density, subjected to dehydration or disrupted to obtain a cell free extract.

With a view to practical use in promoting plant growth, even under adverse conditions such as abiotic stress, promoter agents are usually formulated into compositions also including agriculturally acceptable compounds. Therefore, a sixth aspect the present invention refers to a composition comprising an effective amount of the strain as defined in the first aspect of the invention, the bacterial culture as defined in the second aspect of the invention, or the supernatant of the third aspect of the invention, and one or more agriculturally acceptable compounds.

The term "effective amount" as used herein, means an amount of the strain as defined in the first aspect of the invention, high enough to provide the desired benefit, i.e., the plant growth promotion, but low enough to avoid serious side-effects. The particular dose of the strain administered according to this invention will of course be determined by the particular circumstances surrounding the case, including the compound administered, the route of administration, the particular condition being treated, and the similar considerations.

In an embodiment of the fourth aspect of the invention, optionally in combination with any of the embodiments provided above or below, the strain is present at a concentration from 1.0×10⁵CFUs/mL to 1.0×10¹² CFUs/mL. In another embodiment of the third aspect of the invention, the strain is present at a concentration of 1.0×10⁵ CFUs/mL, 1.0×10⁶ CFUs/mL, 1.0×10⁷ CFUs/mL, 1.0×10⁸ CFUs/mL, 1.0×10⁹ CFUs/mL, 1.0×10¹⁰ CFUs/mL, 1.0×10¹¹ CFUs/mL or 1.0×10¹² CFUs/mL.

For the purposes of the invention, any ranges given include both the lower and the upper end-points of the range.

The amount indicated as CFU/mL relates to the colony forming units (CFU or CFUs) of the strain of the invention per milliliter or gram of the composition.

In one embodiment, the composition comprises the strain CECT30825.

In another embodiment, the composition comprises the strain CECT30286.

In another embodiment, the composition comprises the strains CECT30285 and CECT30286. Advantageously, when both strains were present, a synergistic protective effect was found under saline abiotic stress (see Fig. 4B).

In another embodiment of the sixth aspect of the invention, optionally in combination with any of the embodiments provided above or below, the composition is a phytosanitary composition.

In an embodiment of the sixth aspect of the invention, optionally in combination with any of the embodiments provided above or below, the composition can be in the form of a solution, a pellet, a suspension, a lyophilized composition or other dried compositions (for example freeze dried composition). The lyophilized or dried composition can be reconstituted with a liquid carrier prior to its use or directly used. The composition may be prepared according to various formulations suitable for phytosanitary uses, for example, chosen from the group consisting of formulations of the following type: liquid intended for use without dilution (AL), powder intended for use without dilution (AP), encapsulated granule (CG), contact liquid or gel (CL), contact powder (CP), powdering powder (DP), emulsifiable concentrate (EC), emulsifiable granule (EG), oil type emulsion (EO), water type emulsion (EW), fine granule (FG), macrogranules (GG), emulsifiable gel (GL), powder for spraying (GP), granules (GR), grease (GS), water-soluble gel (GW), microemulsion (ME), microgranules (MG), water-dilutable concentrated suspension (OF), oil dispersion (OD), water-miscible suspension (OL), powder for dispersion in oil (OP), concentrated in gel or paste form (PC), sticks (for agri-pharmaceutical use) (PR), concentrated suspension (SC), suspoemulsion (SE), water-soluble granules (DG), soluble concentrate (SL), film-forming oil (SO), water-soluble powder (SP), water-soluble tablets (ST), tablets (TB), water- dispersible granules (WG), wettable powder (WP), water-dispersible tablets (WT) (the code consisting of two capital letters corresponding to the international codes for phytosanitary formulations).

"Agriculturally acceptable compounds" refers to those compounds and/or materials, which are suitable for use in agriculture. In general, said compounds should be non-toxic to humans and preferably should be environment-friendly.

In a particular embodiment of the sixth aspect of the invention, optionally in combination with any of the embodiments provided above or below, the compositions of the invention may contain compounds for improving the adhesion of the strains in the plants to be treated, as well as phytostrengthener compounds, nutrients, wetting agents, stabilizers, osmotic protectors, antioxidants, sunscreens, buffering compounds or combinations thereof. Examples of adhesion products are gelatin, starch, pectins, alginates and various types of gums such as xanthan. Many of these compounds are also wetting agents. In the case of sunscreens, Congo red, calcium carbonate and wax emulsions can be used. The phytostrengtheners are compounds that can facilitate make crops develop robustness or tolerance towards pathogens or adverse environmental conditions, for example, jasmonic acid analogues and some plant defense stimulants such as harpins, chitosans, and laminarins. Additionally, examples of osmotic protectors are trehalose, betaines and amino acids. Finally, ascorbic acid and glutathione are included among antioxidants. The compositions of the third aspect of the invention can be prepared by routine protocols, such as by mixing the different ingredients.

Another aspect of the present invention provides a cell extract derived from the strain as defined in the first or ninth aspects of the invention, said cell extract being obtainable by a process comprising: (i) inoculating the strain in a suitable culture medium; (ii) subjecting the inoculated culture medium to suitable growth conditions; (iii) separating the cells from the culture medium of step (ii); (iv) collecting the cells and (v) disrupting the cells, (vi) separating the cell extract from cell debris, (vii) collecting the cell extract, and (viii) optionally subjecting the cell extract to a concentration process.

Suitable disrupting means are known by the skilled person and may include physical disruption, for example freeze-thaw or French Press, or chemical disruption, for example by addition of lysozyme. Suitable separation means have been described above. The cell free extract may also be obtained from a cell suspension as defined above, preferably containing metabolite-containing supernatant.

A seventh aspect of the present invention relates to a kit that comprises an effective amount of the strain or mutant thereof, according to the invention, or the bacterial culture of the second aspect of the invention, or the supernatant of the third aspect of the invention, or the composition of the sixth aspect of the invention.

All the embodiments provided above for the strain of the first aspect of the invention, the bacterial culture of the second aspect of the invention, the supernatant of the third aspect of the invention, and the composition of the sixth aspect of the invention are also embodiments of the kit of the seventh aspect of the invention.

In an embodiment of the seventh aspect of the invention, optionally in combination with any of the embodiments provided above or below, the kit may comprise a weekly, monthly, or other periodic dose of the strain of the first aspect of the invention. As an illustrative example, a kit comprising a weekly dose may comprise seven discrete compositions comprising the strain (seven daily doses). As another example, a kit comprising a monthly dose may comprise thirty compositions comprising the strain of the first aspect of the invention.

In case the strain of the first aspect of the invention is lyophilized, the kit of the eighth aspect of the invention can contain a resuspension agent, such as water.

In another embodiment of the seventh aspect of the invention, optionally in combination with any of the embodiments provided above or below, the kit comprises means to facilitate dosing compliance. For example, the kits may be particularly advantageous for the purpose of ensuring that the person who used it is performing the correct administration of the effective amount of the strain of the invention to the plant on the appropriately prescribed schedule. Blister cards or other containing devices appropriately configured may be particularly suitable for clearly illustrating sequence or timing of administration of the various components. The kit may be obtained as one card, or cases of four, six, seven (e.g., a weekly supply), or eight cards co-packaged together. Additionally, monthly or other types of kits may be obtained.

The kit that comprises the isolated strain, or the mutant, of the first aspect of the invention can comprise any means that allows the correct culture of the strain of the invention, such as culture medium, supplements or antibiotics, as well as instructions for the correct preparation and/or application to the plant.

The invention further provides uses and methods for inducing plant growth, increasing abiotic stress tolerance, biomass and yield.

In an embodiment, the strain, the bacterial culture, the supernatant or the composition is applied to plant nursery trays, during seed treating, seed dressing, seed disinfection, seedling root dipping treatment, planting pit treatment, plant foot treatment, planting row treatment, surface spraying, soil incorporation, drip irrigation, or by application to a water culture medium in hydroponic culture.

The term "part of a plant" includes any segment of the plant, such as the root, stem, leaves and seeds.

The term "plant" comprises all plant species cultivated by humans, in particular those intended for food or for animal feed, such as cereals, fodder, vegetable, fruit crops, vines, and/or for the supply of wood for all purposes (such as heating, housing construction furniture, and/or ornamentation). Example of plants include cereals (for example, rice, barley, wheat, rye, oat and corn), beans (for example, soybean, azuki bean, broad bean, peas and peanuts), fruit trees/fruits (for example, apples, pears, citruses, grapes, peaches, apricots, cherries, olive, nuts, almonds, bananas, berries and strawberries), vegetables (for example, tomato, cabbage, spinach, broccoli, lettuce, onion, garlic, leek and pepper), root crops (for example, potato, carrot, sweet potato, radish, lotus root and turnip), industrial crops (for example, cotton, hemp, paper mulberry, mitsumata, rape, beet, hop, sugarcane, sugar beet, rubber, coffee, tobacco, tea), pepos (for example, pumpkin, cucumber, watermelon, melon), pasture plants (for example, orchardgrass, sorghum, Thimothy-grass, clover, alfalfa), lawn grasses (for example, mascarene grass, bentgrass), crops for flavorings (for example, lavender, rosemary, thyme, parsley, basilica, mint, coriander, pepper, ginger), and flower plants (for example, chrysanthemum, rose, orchids).

In another embodiment, the application is performed in the vicinity of the plant or a nursery bed for raising seedlings. Alternatively, in another embodiment the application is performed on the root system.

In the context of the invention, the term "abiotic stress" used herein refers to any adverse effect on metabolism, growth, reproduction and/or viability of a plant. Accordingly, abiotic stress can be induced by suboptimal environmental growth conditions such as, for example, salinity, water deprivation, flooding, low or high temperature, heavy metal toxicity, anaerobiosis, nutrient deficiency, atmospheric pollution or UV irradiation.

As it is shown below, the strains of the invention provide tolerance towards low temperatures and salinity. As shown below, the plants exhibit an increase in weight and number of seeds, which are indicative of the beneficial effects of plant growth and development.

In the context of the invention, the term "abiotic stress tolerance" as used herein refers to the ability of a plant to endure an abiotic stress without suffering a negative alteration in metabolism, productivity and/or viability.

In the context of the invention "chilling temperature" extends from freezing point to, depending on the plant, 7 °C or even 16 °C.

In the context of the invention "freezing temperature" means the temperature at which the any part of the plant freezes.

In one embodiment the strains, compositions and kit of the invention provide tolerance towards temperatures below 5°C, particularly within the range from 5 to -15°C. In one embodiment, the strains, compositions and kit of the invention provide tolerance at a temperature of 5, 4, 3, 4, 1, 0, -1, -2, -3, -4, -5, -6, -7, -8, -9, -10, -11, -12, -13, -14 or - 15°C. As shown below, acclimated tomatoes and rapeseeds, under chilling (4°C) and freezing temperatures, respectively, (-4°C) were able to efficiently grow.

The same occurs when tests were performed to confirm the effect under saline conditions.

Without placing any limitations, plant growth may be in the form of greater root mass, greater depth of rooting, greater shoot mass, greater length of shoots, increased leaf greenness, increased yields, and improved stand and vigor. Plant growth can be established and ascertained by other means besides the extrinsic properties listed above. A person of skill in the art would readily be able to establish physical, biochemical or genetic assays to identify and/or quantify plant growth or viability. The methods of the present invention also describe ways of increasing the yield of crop plants. The strains of the invention can be combined with the crop plant or crop plant seed under conditions effective to colonize the roots of the plant or a plant grown from the plant seed, thereby increasing the yield of the crop plant.

In the context of the invention "biomass" means mass of a plant.

In the context of the invention "Increased yield" means improving the final products produced by plants in a crop, for example, by increasing the number and/or size of fruits, leaves, roots and/or tubers per plant and/or improving the quality of fruits, leaves, roots and/or tubers (e.g. improving flavor, texture, brix, chlorophyll content and/or color).

The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### 1. Bacterial isolation

Bacteria was isolated from rhizomes of *Arabidopsis thaliana* which were grown on natural soil from Collserola, Barcelona, Spain. Roots were washed three times with a solution of 10 mM MgSO₄, followed by one wash with 80% ethanol and 3% sodium hypochlorite in water. Roots were washed three more times with 10 mM MgSO₄ on a rotatory shaker and transferred to 2 ml tubes containing glass beads and 500 µl 10% tryptic soy broth (TSB). Tissue disruption was carried out by mixing at 30 rps for 15 sec. Samples were centrifuged for 3 min at 3000 *g* and the supernatant used for serial dilutions on 20% tryptic soy agar (TSA).

### 2. Characterization of Pararhizobium strains

### 2.1. Phylogenetic analysis

The 16S rRNA gene was amplified with the primers 27F (5'-AGAGTTTGATCCTGGCTCAG-3', SEQ ID NO: 1) and 1492R (5'-GGTTACCTTGTTACGACTT-3', SEQ ID NO: 2). The purified PCR product was sequenced using the primers 27F, 1492R, 337F (5'-GACTCCTACGGGAGGCWGCAG, SEQ ID NO: 3), 799F (5'-AACMGGATTAGATACCCKG, SEQ ID NO: 4), 805R (5'-GACTACHVGGGTATCTAATCC, SEQ ID NO: 5), 1193R (5'-ACGTCATCCCCACCTTCC, SEQ ID NO: 6), and 1492R (5'-GGTTACCTTGTTACGACTT-3', SEQ ID NO: 2).

Reads were assembled into a single contig (**Table 1**) and a BLAST search performed against 16S rRNA EzTaxon database (Kim *et al.*, 2012). 16S rRNA DNA sequences from the bacterial relatives were downloaded from EzTaxon, aligned using MUSCLE algorithm and phylogenetic trees constructed using the maximum-likelihood method based on the Jukes-Cantor model (Kumar et al., 2016). *Pararhizobium* strains 44 and 128 were found in the same clade, as two different related species (99.79% of sequence identity). Other related strains included *Pararhizobium giardinii* H152^{T}, *Pararhizobium polonicum* F5.1^{T}, and *Pararhizobium antarcticum* NAQVI 59^{T} (**Table 2**).

**Table 1. 16S rRNA gene sequences of Pararhizobium strains 44 and 128**

| **P. Strain no.** | **16s rRNA sequence** | **Length (bp)** |
|---|---|---|
| 44 | TCGGTTACCCTTTGTACGACTTCACCCCAGTCGCTGACCC TACCGTGGTTAGCTGCCTCCTTGCGGTTAGCGCACTACCT TCGGGTAAAACCAACTCCCATGGTGTGACGGGCGGTGTGT ACAAGGCCCGGGAACGTATTCACCGCAGCATGCTGATCTG CGATTACTAGCGATTCCAACTTCATGCACTCGAGTTGCAG AGTGCAATCCGAACTGAGATGGCTTTTGGAGATTAGCTCG ACCTCGCGGTCTCGCTGCCCACTGTCACCACCATTGTAGC ACGTGTGTAGCCCAGCCCGTAAGGGCCATGAGGACTTGAC GTCATCCCCACCTTCCTCTCGGCTTATCACCGGCAGTCCC CTTAGAGTGCCCAACTGAATGCTGGCAACTAAGGGCGAGG GTTGCGCTCGTTGCGGGACTTAACCCAACATCTCACGACA CGAGCTGACGACAGCCATGCAGCACCTGTCACCGATCCAG CCTAACTGAAGGACAATGTCTCCACTGTCCGCGATCGGGA TGTCAAGAGCTGGTAAGGTTCTGCGCGTTGCTTCGAATTA AACCACATGCTCCACCGCTTGTGCGGGCCCCCGTCAATTC CTTTGAGTTTTAATCTTGCGACCGTACTCCCCAGGCGGAA TGTTTAATGCGTTAGCTGCGCCACCGAATAGTAAACTACC CGACGGCTAACATTCATCGTTTACGGCGTGGACTACCAGG GTATCTAATCCTGTTTGCTCCCCACGCTTTCGCACCTCAG CGTCAGTAATGGACCAGTGAGCCGCCTTCGCCACTGGTGT TCCTCCGAATATCTACGAATTTCACCTCTACACTCGGAAT TCCACTCACCTCTTCCATACTCTAGATCGACAGTATCAAA GGCAGTTCCAGAGTTGAGCTCTGGGATTTCACCCCTGACT GATCGATCCGCCTACGTGCGCTTTACGCCCAGTAATTCCG AACAACGCTAGCCCCCTTCGTATTACCGCGGCTGCTGGCA CGAAGTTAGCCGGGGCTTCTTCTCCGGATACCGTCATTAT CTTCTCCGGTGAAAGTGCTTTACAACCCTAAGGCCTTCAT CACACACGCGGCATGGCTGGATCAGGCTTGCGCCCATTGT CCAATATTCCCCACTGCTGCCTCCCGTAGGAGTTTGGGCC GTGTCTCAGTCCCAATGTGGCTGATCATCCTCTCAGACCA GCTATGGATCGTCGCCTTGGTAGGCCTTTACCCCACCAAC TAGCTAATCCAACGCGGGCTCATCCATCTCCGATAAATCT TTCCCCCGAAGGGCGTATACGGTATTAGCACAAGTTTCCC TGAGTTATTCCGTAGAGATGGGTAGATTCCCACGCGTTAC | 1446 bp |
| | TCACCCGTCTGCCGCTCCCCTTGCGGGGCGCTCGACTTGC CATGGTTAAGCCTGCCGCCAGCGTTCGTTCTGAGCCATGA TCCAAA (SEQ ID NO: 7) | |
| 128 | GGCTCAGAACGAACGCTGGCGGCAGGCTTAACACATGCAA GTCGAGCGCCCCGCAAGGGGAGCGGCAGACGGGTGAGTAA CGCGTGGGAATCTACCCATCTCTACGGAATAACTCAGGGA AACTTGTGCTAATACCGTATACGCCCTTCGGGGGAAAGAT TTATCGGAGATGGATGAGCCCGCGTTGGATTAGCTAGTTG GTGGGGTAAAGGCCTACCAAGGCGACGATCCATAGCTGGT CTGAGAGGATGATCAGCCACATTGGGACTGAGACACGGCC CAAACTCCTACGGGAGGCAGCAGTGGGGAATATTGGACAA TGGGCGCAAGCCTGATCCAGCCATGCCGCGTGTGTGATGA AGGCCTTAGGGTTGTAAAGCACTTTCACCGGAGAAGATAA TGACGGTATCCGGAGAAGAAGCCCCGGCTAACTTCGTGCC AGCAGCCGCGGTAATACGAAGGGGGCTAGCGTTGTTCGGA ATTACTGGGCGTAAAGCGCACGTAGGCGGATCGATCAGTC AGGGGTGAAATCCCAGAGCTCAACTCTGGAACTGCCTTTG ATACTGTCGATCTAGAGTATGGAAGAGGTGAGTGGAATTC CGAGTGTAGAGGTGAAATTCGTAGATATTCGGAGGAACAC CAGTGGCGAAGGCGGCTCACTGGTCCATTACTGACGCTGA GGTGCGAAAGCGTGGGGAGCAAACAGGATTAGATACCCTG GTAGTCCACGCCGTAAACGATGAATGTTAGCCGTCGGGTA GTTTACTATTCGGTGGCGCAGCTAACGCATTAAACATTCC GCCTGGGGAGTACGGTCGCAAGATTAAAACTCAAAGGAAT TGACGGGGGCCCGCACAAGCGGTGGAGCATGTGGTTTAAT TCGAAGCAACGCGCAGAACCTTACCAGCTCTTGACATCCC GATCGCGGACAGTGGAGACATTGTCCTTCAGTTAGGCTGG ATCGGTGACAGGTGCTGCATGGCTGTCGTCAGCTCGTGTC GTGAGATGTTGGGTTAAGTCCCGCAACGAGCGCAACCCTC GCCCTTAGTTGCCAGCATTCAGTTGGGCACTCTAAGGGGA CTGCCGGTGATAAGCCGAGAGGAAGGTGGGGATGACGTCA AGTCCTCATGGCCCTTACGGGCTGGGCTACACACGTGCTA CAATGGTGGTGACAGTGGGCAGCGAGACCGCGAGGTCGAG CTAATCTCCAAAAGCCATCTCAGTTCGGATTGCACTCTGC AACTCGAGTGCATGAAGTTGGAATCGCTAGTAATCGCAGA TCAGCATGCTGCGGTGAATACGTTCCCGGGCCTTGTACAC ACCGCCCGTCACACCATGGGAGTTGGTTTTACCCGAAGGT AGTGCGCTAACCGCAAGGAGGCAGCTAACCACGGTAGGGT CAGCGACTGGGGTGAAGTCGTACAAAGGGGTAACCGGA (SEQ ID NO: 8) | 1438 bp |

**Table 2. 16S rRNA nucleotide sequence identities between Pararhizobium strains 44, 128 and other phylogenetically related bacterial species.**

| | | | ***Pararhizobium* strain 44** | ***Pararhizobium* strain 128** |
|---|---|---|---|---|
| **Species** | **Strain** | **GenBank identification** | **Sequence identity (%)** | **Sequence identity (%)** |
| *Pararhizobium sp.* | 128 | - | 99.79 | 100 |
| *Pararhizobium sp.* | R129_C | UIBY01000001 | 99.43 | 99.57 |
| *Pararhizobium giardinii* | H152^{T} | ARBG01000149 | 99.43 | 99.57 |
| *Pararhizobium sp.* | OK665 | JQJO01000031 | 99.15 | 99.29 |
| *Pararhizobium sp.* | Root1334 | LMEY01000001 | 99.15 | 99.29 |
| *Pararhizobium polonicum* | F5.1^{T} | LGLV01000030 | 98.44 | 98.58 |
| *Pararhizobium antarcticum* | NAQVI 59^{T} | LSRP01000156 | 97.87 | 98.01 |
| *Pararhizobium sp.* | RAC02 | CP016450 | 97.66 | 97.80 |
| *Rhizobium gei* | ZFJT-2^{T} | KF551166 | 98.26 | 98.40 |
| *Rhizobium aquaticum* | SA-276^{T} | KM083136 | 97.45 | 97.59 |
| *Pararhizobium herbae* | CCBAU 83011^{T} | GU565534 | 99.47 | 99.47 |
| *Rhizobium daejeonense* | KCTC 12121^{T} | AY341343 | 97.37 | 97.51 |
| *Ensifer adhaerens* | Casida A^{T} | JNAE01000171 | 97.23 | 97.37 |
| *Pararhizobium sp.* | Root482 | LMFA01000020 | 97.17 | 97.31 |
| *Ensifer medicae* | WSM419^{T} | CP000738 | 97.02 | 97.16 |
| *Pararhizobium sp.* | Root1212 | LMDA01000001 | 96.95 | 97.09 |
| *Ciceribacter thiooxidans* | F43b^{T} | KU975391 | 96.89 | 97.03 |
| *Rhizobium selenitireducens* | ATCC BAA-1503^{T} | JAEG01000027 | 96.89 | 97.03 |
| *Ensifer morelensis* | Lc04^{T} | AY024335 | 96.88 | 97.02 |
| *Ensifer meliloti* | LMG 6133^{T} | X67222 | 96.81 | 96.95 |
| *Ensifer psoraleae* | CCBAU 65732^{T} | EU618039 | 97.07 | 97.21 |
| *Ensifer arboris* | LMG 14919^{T} | ATYB01000014 | 96.74 | 96.88 |
| *Ensifer numidicus* | ORS 1407^{T} | AY500254 | 96.74 | 96.88 |
| *Ensifer americanus* | CFNEI 156^{T} | LNQC01000019 | 96.67 | 96.81 |
| *Ensifer fredii* | NBRC 14780^{T} | BJNI01000207 | 96.67 | 96.81 |
| *Rhizobium sp.* | TH135 | PKRP01000013 | 96.53 | 96.67 |
| *Rhizobium naphthalenivora ns* | TSY03b^{T} | AB663504 | 96.53 | 96.67 |
| *Ensifer kostiensis* | LMG 1922^{T}7 | AM181748 | 96.79 | 96.93 |
| *Hoeflea anabaenae* | WH2K^{T} | DQ364238 | 96.52 | 96.66 |
| *Rhizobium azooxidifex* | Po 20/26^{T} | LN832063 | 96.84 | 96.98 |
| *Ensifer garamanticus* | ORS 1400^{T} | AY500255 | 96.45 | 96.59 |
| *Rhizobium rhizogenes* | NBRC 13257 | BAYX01000035 | 96.45 | 96.59 |
| *Ensifer sesbaniae* | CCBAU 65729^{T} | JF834143 | 96.97 | 97.04 |
| *Ensifer aridi* | LMR001^{T} | LUAV01000069 | 96.39 | 96.53 |
| *Rhizobium lusitanum* | P1-7^{T} | jgi.1052907 | 96.38 | 96.52 |
| *Rhizobium cauense* | CCBAU 101002^{T} | JQ308326 | 96.39 | 96.53 |
| *Rhizobium sp.* | CAAS-2R | FJ793198 | 96.32 | 96.46 |
| *Agrobacterium fabacearum* | CNPSo 675^{T} | MN741112 | 96.31 | 96.45 |
| *Agrobacterium arsenijevicii* | KFB 330^{T} | JWIT01000061 | 96.31 | 96.45 |
| *Rhizobium dioscoreae* | S-93^{T} | LC498520 | 96.32 | 96.46 |
| *Agrobacterium nepotum* | 39/7^{T} | JWJH01000079 | 96.24 | 96.38 |
| *Rhizobium subbaraonis* | JC85^{T} | OBQD01000060 | 96.24 | 96.38 |
| *Rhizobium rosettiformans* | W3^{T} | EU781656 | 96.69 | 96.83 |
| *Agrobacterium radiobacter* | ATCC 19358^{T} | AJ389904 | 96.17 | 96.31 |
| *Rhizobium sp.* | Root1220 | LMDG01000001 | 96.18 | 96.32 |
| *Ensifer kummerowiae* | CCBAU 71714^{T} | AY034028 | 96.10 | 96.24 |
| *Rhizobium sphaerophysae* | CCNWGS 0238 | FJ154088 | 96.40 | 96.54 |
| *Rhizobium rhizophilum* | 7209-2^{T} | KX017286 | 96.64 | 96.78 |
| *Pararhizobium capsulatum* | IFAM 1004^{T} | X73042 | 96.61 | 96.61 |
| *Rhizobium mayense* | CCGE526^{T} | JX855172 | 96.37 | 96.37 |
| *Rhizobium calliandrae* | CCGE524^{T} | JX855162 | 96.22 | 96.22 |
| *Rhizobium tubonense* | CCBAU 85046^{T} | EU256434 | 96.42 | 96.49 |
| *Rhizobium mesoamericanu m* | CCGE 501^{T} | JF424606 | 96.39 | 96.39 |
| *Rhizobium esperanzae* | CNPSo 668^{T} | KC293513 | 96.31 | 96.31 |
| *Pseudomonas fitomaticsae* | FIT81^{T} | MZ773500.1 | 83.49 | 83.64 |

### 2.2. Bacterial description

Growth of *Pararhizobium* strain 44 required 24 h when the bacterium was cultured in TSB media and up to 96 h when cultured in Yeast Extract Mannitol (YEM; 0.5 g/L of yeast extract, 5 g/L of mannitol, 0.5 g/L MgSO₄*7H₂O, 0.1 g/L NaCl, pH 7.0). The bacterium was identified as rod-shaped by microscopy and Gram-negative by staining. Catalase activity was determined by the production of O₂ using 3% hydrogen peroxide as substrate. The absence of oxygen production indicated the absence of catalase activity. Cytochrome C oxidase activity was tested using a colorimetric assay in the presence of 1% (w/v) tetramethyl-p-phenylenediamine dihydrochloride. The results were positive for Cytochrome C oxidase activity.

For transmission electron microscopy (TEM) cells were adsorbed on a carbon-coated copper grid for 1 min and then, washed with milliQ water for 1 min. Next, it was negatively stained by floating the grid on a drop of 2% (w/v) uranyl acetate in water for 1 min. The excess of liquid was removed manually from the edge of the grids. The sample was observed under a transmission electron microscope Jeol J1010 (Gatan, Japan) equipped with a tungsten filament. Images were acquired at 80 kV using a CCD Megaview camera (1k × 1k pixels). The acquired images were used to determine an average cell size of -2.4 ± 0.2 µm in length and ∼1.2 ± 0.1 µm in width. Temperature tolerance was determined by culturing 200 µl of a bacterial suspension (OD₆₀₀=0.01) in YEM media up to 28 days at the following temperatures: 4 °C, 10 °C, 15 °C, 20 °C, 25 °C, 28 °C, 30 °C, 35 °C, 36 °C, 37 °C and 40 °C. The *Pararhizobium* strain 44 could grow between 4 °C to 35 °C, with an optimal growth at 28 °C. Salt tolerance of the *Pararhizobium* strain 44 was assessed by culturing 50 µl of a bacterial suspension (OD₆₀₀=0.01) of strain 44 in 2 ml of YEM media supplemented with up to 9% (w/v) NaCl, followed by incubation at 28 °C shaking (190 rpm) for up to 28 days. The strain 44 demonstrated the ability to grow in the presence of up to 9% NaCl.
pH tolerance of the *Pararhizobium* strain 44 was tested by culturing 200 µl of the bacterial suspension (OD₆₀₀=0.01 ) in phosphate buffered YEM media at pH 5.0, pH 6.0, pH 7.0, pH 8.0, and pH 9.0. The *Pararhizobium* strain 44 could grow between pH 5.0 to pH 9.0.

Growth of *Pararhizobium* strain 128 required 24 h when the bacterium was cultured in TSB media and up to 96 h when cultured in YEM media. The bacterium was identified as rod-shaped by microscopy and Gram-negative by staining. Catalase activity was determined by the production of O₂ using 3% hydrogen peroxide as substrate. The absence of oxygen production indicated the absence of catalase activity. Cytochrome C oxidase activity was tested using a colorimetric assay in the presence of 1% (w/v) tetramethyl-p-phenylenediamine dihydrochloride. The results were positive for Cytochrome C oxidase activity.

For TEM visualization of the *Pararhizobium* strain 128, the same protocol as described above was used. The acquired images were used to determine an average cell size of ∼1.9 ± 0.2 µm in length and ∼0.8 ± 0.1 µm width. Temperature tolerance was determined as described above. The *Pararhizobium* strain 128 could grow between 4 to 36 °C, with an optimal growth at 28°C. Salt tolerance of the *Pararhizobium* strain 128 was evaluated as described above. The strain demonstrated the ability to grow in the presence of up to 9% NaCl. The pH tolerance of the *Pararhizobium* strain 128 was determined as described above. The strain could grow between pH 5.0 to pH 10.0.

### 2.3. Biochemical characterization of bacteria

### 2.3.1. Carbon source utilization. Chemical tolerance.

Carbon source utilization was assessed using the Genlll Microplate (Biolog, Hayward, USA), following the manufacturer's instructions. Briefly, cell suspension of the bacteria is inoculated into the GEN III MicroPlate, 100 µl per well, and the MicroPlate is incubated at 28 °C to allow the phenotypic fingerprinting. All the wells start out colorless when inoculated. Increased respiration in the wells where cells can utilize a carbon source and/or grow causes reduction of a tetrazolium redox dye, forming a purple color (positive result). Negative wells remain colorless (negative result). Slightly colored wells may be due to weak carbon source utilization.

Table 3 summarizes the results of this analysis.

**Table 3. Biochemical characterization of carbon source utilization in Pararhizobium strains 44 and 128. Results are indicated as positive (+), negative (-) or weak (w), depending on the ability to utilize and/or grow under the different carbon sources.**

| | *Pararhizobium* strain 44 | *Pararhizobium* strain 128 |
|---|---|---|
| Dextrin | + | + |
| D-Maltose | + | + |
| D-Trehalose | + | + |
| D-Cellobiose | + | + |
| Gentiobiose | + | + |
| Sucrose | + | + |
| D-Turanose | + | + |
| Stachyose | - | - |
| D-Raffinose | + | - |
| α-D-Lactose | + | - |
| D-Melibiose | + | - |
| β-Methyl-D-glucoside | + | - |
| D-Salicin | + | - |
| N-Acetyl-D-glucosamine | + | + |
| N-Acetyl-β-D-mannosamine | + | + |
| N-Acetyl-D-galactosamine | + | w |
| N-Acetyl neuraminic acid | - | - |
| α-D-Glucose | + | + |
| D-Mannose | + | + |
| D-Fructose | + | + |
| D-Galactose | + | + |
| 3-Methyl glucose | - | + |
| D-Fucose | + | + |
| L-Fucose | + | + |
| L-Rhamnose | + | + |
| Inosine | + | - |
| D-Sorbitol | + | - |
| D-Mannitol | + | - |
| D-Arabitol | + | + |
| myo-Inositol | + | - |
| Glycerol | + | + |
| D-Glucose-6-phosphate | + | w |
| D-Fructose-6-phosphate | w | w |
| D-Aspartic acid | - | - |
| D-Serine | - | - |
| Gelatin | - | - |
| Glycyl-L-proline | + | - |
| L-Alanine | w | - |
| L-Arginine | - | - |
| L-Aspartic acid | w | w |
| L-Glutamic acid | w | w |
| L-Histidine | - | w |
| L-Pyroglutamic acid | + | + |
| L-Serine | - | - |
| Pectin | + | + |
| D-Galacturonic acid | + | + |
| L-Galactonic acid lactone | w. | w |
| D-Gluconic acid | + | + |
| D-Glucuronic acid | + | + |
| Glucuronamide | + | + |
| Mucic acid | - | + |
| Quinic acid | w | + |
| D-Saccharic acid | - | + |
| p-Hydroxyphenylacetic acid | - | - |
| Methyl pyruvate | - | + |
| D-Lactic acid methyl ester | + | + |
| D-Lactic acid | + | + |
| Citric acid | - | - |
| α-Keto-glutaric acid | - | + |
| D-Malic acid | - | + |
| L-Malic acid | w | + |
| Bromo-succinic acid | - | w |
| Tween 40 | + | + |
| γ-Aminobutyric acid | w | - |
| α-Hydroxybutyric acid | - | - |
| β-Hydroxy-D,L-butyric acid | w | - |
| α-Ketobutyric acid | - | - |
| Acetoacetic acid | - | + |
| Propionic acid | - | - |
| Acetic acid | - | + |
| Formic acid | w | - |
| pH5 | + | + |
| pH10 | - | + |
| 1% NaCl | + | + |
| 4% NaCl | + | + |
| 8% NaCl | + | + |
| 9% NaCl | + | + |
| 1% Sodium lactate | + | + |
| Fusidic acid | + | + |
| D-Serine | + | + |
| Troleandomycin | + | + |
| Rifamycin SV | + | + |
| Minocycline | + | + |
| Lincomycin | + | + |
| Guanidine HCl | + | + |
| Niaproof 4 | + | + |
| Vancomycin | + | + |
| Tetrazolium violet | + | + |
| Tetrazolium blue | + | + |
| Nalidixic acid | + | + |
| Lithium chloride | + | + |
| Potassium tellurite | + | + |
| Aztreonam | + | + |
| Sodium butyrate | + | + |
| Sodium bromate | + | + |
| Carbenicillin | + | + |
| Rifampicin | + | + |
| Gentamicin | + | - |
| Streptomycin | + | + |
| Spectinomycin | + | - |
| Cefotaxime | + | + |
| Kanamycin | + | - |

### 2.3.2. Enzymatic activities

The enzymatic activities of *Pararhizobium* strains 44 and 128 were determined using the API Zym strips, according to the manufacturer's instructions (bioMérieux, Marcy-I'Étoile, France).

The results are summarized in **Table 4** below:

**Table 4. API Zym tests for assessing the enzymatic activities of Pararhizobium strains 44 and 128. Results are indicated as positive (+) or negative (-).**

| | *Pararhizobium* strain 44 | *Pararhizobium* strain 128 |
|---|---|---|
| Alkaline phosphatase | + | + |
| C4 esterase | + | + |
| C8 esterase | + | + |
| C14 lipase | + | - |
| Leucine arylamidase | + | + |
| Valine arylamidase | + | + |
| Cystine arylamidase | + | - |
| Trypsin | + | + |
| α-chemotrypsin | + | - |
| Acid phosphatase | + | + |
| Naphthol-AS-BI-phosphohydrolase | + | + |
| α-galactosidase | - | - |
| β-galactosidase | + | - |
| β-glucuronidase | - | - |
| α-glucosidase | + | + |
| β-glucosidase | + | - |
| N-acetyl-β-glucosaminidase | + | + |
| α-mannosidase | - | - |
| α-fucosidase | - | - |

### 2.3.3. Determination of cellular fatty acids and polar lipids

Polar lipids (PL) and cellular fatty acids (CFA) were determined in four-day-old bacteria cultivated in YEM media and freeze-dried. The analyses were performed by the Deutsche Sammlung von Mikroorganismen und Zelkulturen (DSMZ) at the Leibniz Institute (Braunschweig, Germany)

CFA were analyzed after conversion of fatty acids into fatty acid methyl esters (FAMEs) by saponification, methylation and extraction according to (Sasser, 1990). The fatty acid methyl esters mixtures were separated by gas chromatography and detected by a flame ionization detector. In subsequent analyses, fatty acids were identified by a GC-MS run, on an Agilent GC-MS 7000D system (Vieira *et al.,* 2021). Peaks were identified based on retention time and mass spectra. The position of single double bounds was confirmed by derivatization to the corresponding dimethyl disulfide adduct (Moss and Lambert-Fair, 1989). Branched-chain fatty acid positions, cyclo-positions and multiple double bounds were determined by derivatization to their 3-pyridylcarbinol and/or 4,4-dimethyloxazoline (DMOX) derivatives (Harvey, 1982; Spitzer, 1996; Yu *et al.,* 1988).

The composition of CFA in *Pararhizobium* strains 44 and 128 is shown in Table 5 below.

**Table 5. Composition of cellular fatty acids in Pararhizobium strains 44 and 128. Values below 1% are not shown.**

| | *Pararhizobium* strain 44 | *Pararhizobium* strain 128 |
|---|---|---|
| C_{14:0} 3-OH | 5.1% | 3.6% |
| C_{16:0} | 7.6% | 20.2% |
| C_{16:0} 3-OH | 1% | 1.1% |
| C_{17:0} cyclo w7c | 3.8% | 4.1% |
| C_{18:0} | 2% | 5.5% |
| C_{18:0} 3-OH | 2% | 1.7% |
| C_{18:1} w7c 11-methyl | 24.8% | 19.9% |
| C_{18:1} w7c | 41.8% | 27.7% |
| C_{19:0} cyclo w7c | 5% | 1.7% |
| Summed feature 3 (C_{16:1} w7c/C_{16:1} w6c) | 6.6% | 4.2% |

PL were extracted using chloroform:methanol:0.3% NaCl (1:2:0.8 v/v/v). Polar lipids were recovered into the chloroform phase and separated by two-dimensional thin layer chromatography. The mobile phase of the first dimension was chloroform/ methanol/water (65:25:4 v/v/v); the mobile phase of the second dimension was chloroform/methanol/acetic acid/ water (80:12:15:4 v/v/v/v). Total lipid material was detected using molybdatophosphoric acid and specific functional groups detected using spray reagents specific for defined functional groups (Tindall *et al.,* 2007).

The main PL for *Pararhizobium* strain 44 were phosphatidylcholine, phosphatidylethanolamine, phosphatidylglycerol, aminophospholipid, aminolipid, and phospholipid.

The main PL for *Pararhizobium* strain 128 were phosphatidylcholine, phosphatidylethanolamine, phosphatidylglycerol, aminophospholipid, aminolipid, glycolipid, and phospholipid.

### 2.3.4. Deposit in an International Depositary Authority recognized under the Budapest Treaty

*Pararhizobium* strains 44 and 128, isolated from an agricultural soil collected in Collserola, Barcelona (Spain), *were deposited on April 25, 2023 at the* Colección Española de Cultivos Tipo (CECT) in the Universidad de Valencia 46100 Burjasot, Valencia (Spain), by Universitat de Barcelona, sited at Gran Via de les Corts Catalanes, 585, Barcelona (Spain).

The strains, identified by the depositor with the references "44" and "128", received the CECT accession numbers CECT 30825 and CECT 30826, respectively, after the International Authority of Deposit declared the strain as viable.

### 3. Tests performed with the strains of the invention

### 3.1. Plant materials, preparation of bacteria suspensions and inoculation procedures

Tomato *(Solanum lycopersicum)* plants var. "Moneymaker" were used in this study. Rapeseed *(Brassica napus)* plants var. "Valle de Oro" were used in this study. Cultivation of plants for stress treatments was performed in temperature and light-regulated growth chambers. For the determination of growth and yield parameters, tomato plants were grown in a glass greenhouse, and rapeseed plants were grown outdoor. In all cases, plant cultivation was performed at the facilities of the "Servei de Camps Experimentals" of the Universitat de Barcelona (Av. Diagonal, 643. 08028 Barcelona (Spain)).

For the preparation of bacteria suspensions, strains 44 and 128 were inoculated in YEM media (0.5 g/L of yeast extract, 5 g/L of mannitol, 0.5 g/L MgSO₄*7H₂O, 0.1 g/L NaCl, pH 7.0) and incubated by shaking at 240 rpm and 28 °C for 48 h. The cell culture was centrifuged at 6000 *g* for 15 min, and the bacterial pellet washed two times with 100 ml sterile water. The bacteria were then resuspended in sterile water to a final concentration of 10⁸ CFU/ml.

Spray inoculation with the bacterial suspension (10⁸ CFU/ml) was performed using a pressure sprayer to an average of 2 ml per plant (at seedling stage) or 10 ml per plant (at adult stage).

Soil inoculation was performed by watering the pots with 20 ml of the bacterial suspension (10⁸ CFU/ml). Mock inoculation was performed with sterile water.

### 3.2. Chilling and freezing stress treatments

Tomato *(Solanum lycopersicum* var. Moneymaker) seeds were sown on soil containing a mixture of peat moss (40%), vermiculite (50%) and perlite (10 %). Plants were grown in growth chambers at 20-22°C under 16 h light / 8 h dark cycles, 200 µmol photons m⁻² s⁻¹ of light intensity and 70 % relative humidity. Treatments with *Pararhizobium* strains 44 and 128 were performed by spray-inoculation of a bacterial suspension containing 10⁸ CFU/ml in water on day 7 and 14 after germination. Mock-inoculated plants were treated with water. On day 18, tomato plants were exposed to 4 °C for three days under the same photoperiod conditions in the same growth cabinet. Afterwards, plants were incubated at 22 °C for seven additional days for recovery.

Rapeseed seeds were germinated directly on soil containing a mixture of peat moss (40%), vermiculite (50%) and perlite (10 %). Plants were grown at 20-22°C under 16 h light / 8 h dark cycles at 100-125 µmol photons m⁻² s⁻¹ of light intensity and 70 % relative humidity. Treatments with *Pararhizobium* strains 44 or 128 were performed by spray or soil-inoculation of a bacterial suspension containing 10⁸ CFU/ml in water on day 7 and 14 after germination. Mock-inoculated plants were treated with water. On day 21, plants were exposed to 4 °C for seven days for cold-acclimation. On day 28, plants were exposed to - 4 °C for 16 h, followed by incubation at 22 °C for seven additional days. The survival rates were calculated by the ratio of plants that resumed growth divided by the total number of plants in each treatment.

### 3.3. Salt stress treatments

For the determination of survival rates to salt stress, rapeseed plants were germinated on growth media (half-strength Murashige and Skoog salts (MS) supplemented with vitamins (Duchefa Biochemie), 1% sucrose, 0.6% plant agar and 0.05 % MES adjusted to pH 5.7) containing 300 mM NaCl and 10⁸ CFU/ml of *Pararhizobium* strains 44, 128, the combination of both (44 + 128) or mock (no bacteria). Seedlings were exposed to saline conditions for three weeks. Survival rates were determined by the number of plants that resumed growth after a 7-day recovery in half-strength MS media without NaCl.

For the determination of the reative electrolyte leakage (REL) and polyamine levels under salinity, rapeseed plants were germinated and grown on soil containing a mixture of peat moss (40%), vermiculite (50%) and perlite (10 %) at 20-22°C under 16 h light / 8 h dark cycles. 200 µmol photons m⁻² s⁻¹ of light intensity and 70 % relative humidity. Plants were spray-inoculated with 10⁸ CFU/ml of *Pararhizobium* strains 44, 128, a combination of both (44 + 128) or mock (water) on day 7 and 14 after germination. Irrigation with 250 mM NaCl was imposed from day 18 to 28 after germination.

### 3.4. Determination of relative electrolyte leakage

To measure electrolyte leakage, we selected at least 10 plants and detached five leaf discs from each using a 0.5 cm diameter cork borer. The leaf discs were submerged in 10 ml of distilled water and gently agitated for 10 minutes to remove any extraneous ions. Subsequently, the leaf discs were acclimated for 1 h at room temperature to reach a steady state before conducting the measurements. The initial conductivity of the samples was recorded using a calibrated electrode-based conductometer (Mettler Toledo, Columbus, USA). After recording the initial conductivity, the leaf discs were boiled for 20 min in distilled water and then cooled down to room temperature. Finally, the final conductivity was measured. Ion leakage was determined by calculating the ratio of initial conductivity to final conductivity and was expressed as a percentage. To ensure accuracy, conductivity measurements were taken at least three times for each sample.

### 3.5. Determination of fresh weight (FW), dry weight (DW) and relative water content (RWC)

The fresh weight (FW) was determined by direct sampling of plants and weighting on an analytical balance. The dry weight (DW) was determined by weighing the plants after removing their water content by incubation at 80 °C for 48 h. To measure Relative Water Content (RWC) in plant tissues, leaves were weighed to determine the fresh weight (FW). The leaves were then incubated in distilled water overnight to reach full hydration. After 24 h, the leaf surfaces were dry using paper towel and the turgid weight (TW) determined. Next, samples were dry in an oven at 80°C for 48 h and the dry weight (DW) determined. RWC was calculated using the formula [(FW-DW)/(TW-DW)] × 100%.

### 3.6. Determination of lipid peroxidation

A lipid peroxidation assay kit (Sigma-Aldrich, St. Louis, USA) was used for the quantification of MDA according to manufacturer's instructions. Briefly, 10 mg of fresh homogenized plant tissue was mixed with 300 µl of MDA lysis buffer containing butylated hydroxytoluene (BHT). The samples were centrifuged at 13000 *g* for 10 min, and the resulting supernatant was transferred to a new tube. To this, 600 µl of thiobarbituric acid (TBA) was added, and the mixture was incubated at 95 °C for 1 h. Following this, the mixture was cooled down to room temperature. The MDA-TBA adduct was quantified by measuring the absorbance at 532 nm using a Multiskan plate reader (Thermo Fisher Scientific Inc, Waltham, USA). A standard curve was generated using known concentrations of MDA, and the concentration of MDA in the samples was determined based on this curve.

### 3.7. Determination of polyamine levels

Polyamines were derivatized with dansyl chloride and analyzed by high-performance liquid chromatography (HPLC) according to the method described by (Marcé *et al.,* 1995). Briefly, 100 mg of fresh homogenized plant material were mixed with 1 ml 5% (v/v) perchloric acid. Samples were incubated on ice for 5 min and centrifuged for 10 min at 16000 *g* at 4 °C. The supernatant was transferred to a new tube and mixed with a saturated solution of sodium carbonate and dansyl chloride (5 mg/ml). Dansylated polyamines were recovered with toluene, dried under speed-vac, and resuspended in 0.8 ml acetonitrile. Polyamines were analyzed using HPLC. The chromatography was performed using a C18 reverse-phase column and a mobile phase consisting of acetonitrile and water (70:30, v/v). The fluorescence of the dansylated polyamines was detected using an excitation wavelength of 252 nm and an emission wavelength of 500 nm.

### 4. Results

### 4.1. Freezing and cold stress tolerance assays

### 4.1.1. Effect of the inoculation with Pararhizobium strains 44, 128 and their combination on cold stress tolerance in tomato

To evaluate the effect of the inoculation with the *Pararhizobium* strains 44, 128 and the combined 44+128 on cold stress tolerance in tomato, four-week-old tomato (var. Moneymaker) plants grown at 22 °C and inoculated with the corresponding strains or mock, were exposed for three days at 4 °C. After this period, relative electrolyte leakage (REL) was determined in leaves (**Fig. 1A**). The degree of ion leakage is considered as a measure of the level of cellular damage, with higher levels of electrolyte leakage indicating greater damage to the cell membrane. The tomato plants inoculated with the *Pararhizobium* strains 44 or 128 exhibited significantly less electrolyte leakage than the mock (M, water) treatment (**Fig. 1A**). At the end of the cold treatment (3 d.), the fresh and dry weights of the tomato plants were not significantly different between the *Pararhizobium* treatments and the mock **(****Fig. 1B and 1C****).** However, after 7 days of recovery (7 d.p.r) at 22 °C, the tomato plants inoculated with *Pararhizobium* strain 44 resumed growth better and exhibited significantly higher fresh and dry weight compared to the mock treatment **(****Fig. 1B and 1C****).** The data indicated that *Pararhizobium* strains 44 and 128 alleviate the cellular damage imposed by cold stress. In addition, the treatment with the *Pararhizobium* strain 44 improved cold stress post-recovery in tomato.

To further analyze the effect of the treatments with *Pararhizobium* strains on cold stress, we measured the levels of polyamines, which are known stress-protective compounds (Alcázar et al., 2020), at the beginning (0 d.) and at 3 days (3 d.) of cold (4 °C) treatment in tomato plants previously inoculated with the *Pararhizobium* strain 44, 128, 44+128 or mock (M, water) (**Fig. 2A**). Before the stress treatment, the levels of Putrescine (Put) in the tomato plants spray-inoculated with *Pararhizobium* strain 44 were similar to the mock treatment. Interestingly, plants treated with *Pararhizobium* strain 44 exhibited two-fold higher increases of Put in response to cold, than those treated with mock (**Fig. 2A**). The concentration of Spermine (Spm) at 0 d. was significantly higher in plants treated with the combined *Pararhizobium* strains 44+128 than plants treated with mock. The combined 44+128 treatment also increased the Spm content of cold-stressed tomato plants at 3 d. compared to the mock control (**Fig. 2A**). Overall, the inoculation with *Pararhizobium* strains 44 and combined 44+128 led to increased polyamine biosynthesis in response to cold stress in tomato.

MDA levels have been widely used as a biomarker of oxidative stress and lipid peroxidation in plants. Low MDA levels are correlated with increased capacity to scavenge reactive oxygen species (ROS) and prevent lipid peroxidation (Davey *et al.,* 2005). The inoculation of tomato plants with *Pararhizobium* strains 44 or 128 led to significantly lower concentration of MDA compared to the mock treatment after three days of cold stress (3 d.) (**Fig. 2B**). The data indicated that treatment with *Pararhizobium* strains 44 or 128 prevents oxidative stress and lipid peroxidation during cold exposure in tomato.

### 4.1.2. Effect of the inoculation with Pararhizobium strains 44, 128 and combined 44+128 on freezing tolerance in rapeseed

We analyzed the effect of spray or soil-inoculation with *Pararhizobium* strains 44 or 128 on freezing stress tolerance in rapeseed (**Fig. 3**). For this, rapeseed plants germinated and grown on soil at 22 °C were spray or soil- inoculated with *Pararhizobium* strains 44, 128 or mock (M, water) after 7 and 14 days of germination. Rapeseed plants were then exposed to 4 °C from day 21 to 28 to simulate natural acclimation to cold. On day 28, the plants were exposed to harmful freezing conditions (- 4 °C) for 16 h, and survival rates (S (%)) determined after 7 days of recovery at 22 °C.

Spray and soil-inoculation with *Pararhizobium* strains 44 or 128 increased the survival rate of rapeseed plants to freezing temperature (- 4 °C) by almost two-fold **(****Figures 3A and 3B****).** These results indicated that the *Pararhizobium* strains 44 and 128 facilitate cold acclimation and improve freezing tolerance in rapeseed.

### 4.2. Salt stress tolerance assays

### 4.2.1. Effect of Pararhizobium strains 44 and 128 on salt stress tolerance in rapeseed

To evaluate the effect of the inoculation with the *Pararhizobium* strains 44, 128 and the combination of both (44 + 128) on salinity stress tolerance in rapeseed, we determined the survival rates (S (%)) of rapeseed seedlings germinated and grown under *in vitro* conditions for three weeks on half-strength Murashige and Skoog's media supplemented with 300 mM NaCl. The salt stress treatment led to a significant reduction in seedling viability to less than 15% in mock (M) inoculated plants (**Fig. 4A**). The individual treatments with *Pararhizobium* strains 44 or 128 increased the survival rates to salt stress more than twice the mock treatment. Interestingly, the combined treatment with *Pararhizobium* strains 44+128 produced a stronger response than the individual treatments alone (**Fig. 4A**). To further investigate the effect of the different *Pararhizobium* strains on salinity tolerance in rapeseed plants grown on soil, we determined the relative electrolyte leakage (REL) and relative water content (RWC) in 4-week-old seedlings spray-inoculated with *Pararhizobium* strains 44, 128, combined 44+128 or mock, and irrigated with 250 mM NaCl for ten days **(****Fig. 4B** **and Table 6).** The treatment with *Pararhizobium* strains 44 and 128 led to a significant reduction in the REL in response to salinity, which was stronger in the combined *Pararhizobium* 44+128 treatment (**Fig. 4B**). Consistent with these results, the RWC was higher in the inoculations with *Pararhizobium* strains 44, 128 and 44+128 than in the mock (water) inoculation under saline conditions **(Table 6).** The data indicated that inoculation with the *Pararhizobium* strains 44 and 128 enhances salt stress tolerance in rapeseed and the combined inoculation with *Pararhizobium* strains 44 and 128 produced a stronger protective response.

**Table 6.** Relative water content (RWC) of rapeseed plants spray-inoculated with *Pararhizobium* strains 44, 128, the combination of both (44 + 128) or mock (water) after 10 days of irrigation with 250 mM NaCl. Rapeseed plants were germinated and grown on soil and inoculated with 10⁸ CFU/ml of *Pararhizobium* strains 44, 128, a combination of both (44 + 128) or mock (water) on day 7 and 14 after germination. Irrigation with 250 mM was imposed from day 18 to 28 after germination. RWC was determined on day 28 after germination. The values represent the mean ± standard deviation (S.D.) from ten biological replicates per treatment.

| | **RWC (%) ± S.D.** | |
|---|---|---|
| **Treatment** | **0 mM NaCl** | **250 mM NaCl** |
| Mock | 90 ± 2.1 | 40 ± 4.2 |
| *Pararhizobium* strain 44 | 88 ± 3.2 | 60 ± 3.1 |
| *Pararhizobium* strain 128 | 87 ± 2.5 | 59 ± 2.0 |
| *Pararhizobium* strains 44 + 128 | 90 ± 1.5 | 75 ± 2.3 |

To further analyze the effect of the inoculation with the *Pararhizobium* strains on salinity stress tolerance in rapeseed, we determined the concentration of polyamines before the salt stress treatment (0 d.) and after 10 days (10 d.) of salt stress (250 mM NaCl). Before the salt stress, rapeseed plants inoculated with *Pararhizobium* strains 44+128 contained similar Put levels to mock (M, water) inoculated plants (**Fig. 5**). However, after 10 days of salt stress they exhibited significantly higher Put levels than mock-inoculated plants (Fig. 5). The levels of Spd and Spm were significantly higher in the treatments with the *Pararhizobium* strains 44, 128 and the combination of both (44+128) under saline conditions (**Fig. 5**). The data indicated that treatment with *Pararhizobium* strains 44, 128 and their combination (44+128) stimulates the biosynthesis of Spd and Spm during salt stress in rapeseed, while the combination of *Pararhizobium* strains 44+128 was also effective in increasing Put levels.

### 5. Yield performance

### 5.1. Yield performance of tomato plants inoculated with Pararhizobium strains 44 and 128

We determined tomato yield performance and fruit quality traits in plants inoculated with *Pararhizobium* strains 44, 128 and mock (M, water) by measuring the weight per fruit (WPF), total number of fruits per plant (FN), pH, titratable acidity (TA), total soluble solid (TSS or °Brix) and the ratio between TSS and TA at the ripening red stage (**Fig. 6**). The inoculation with *Pararhizobium* strains 44 and 128 led to a significant increase in fruit weight (**Fig. 6A**) but not increased number of fruits per plant (**Fig. 6B**). The pH (**Fig. 6C**) and TSS (° Brix) content (**Fig. 6E**) did not differ between tomato fruits obtained from mock and *Pararhizobium*-inoculated plants. In contrast, inoculation with *Pararhizobium* strains 44 and 128 significantly decreased the TA (**Fig. 6D**), thus leading to higher TSS/TA ratio (**Fig. 6F**).

### 5.2. Yield performance of rapeseed plants inoculated with Pararhizobium strains 44 and 128

To compare the yield performance of rapeseed plants inoculated with the *Pararhizobium* strains 44 and 128 with the mock treatment, we determined the seed weight per plant (SW) (Fig. 7A), weight per 100 seeds (W) (Fig. 7B) and stem length (SL) (Fig. 7C) of rapeseed plants inoculated with the respective treatments. The inoculation with *Pararhizobium* strains 44 and 128 significantly increased the seed weight per plant by 1.3-fold the mock (M, water) treatment (**Fig. 7A**)and led to a slight but significant increase in stem length at ripening stage (**Fig. 7C**). The weight per seed did not show significant differences between mock and *Pararhizobium-*inoculated plants (**Fig. 7B**). The data indicated that treatment of rapeseed plants with *Pararhizobium* strains 44 and 128 increased seed yield.

### REFERENCES

Alcázar, R., Bueno, M. and Tiburcio, A.F. (2020) Polyamines: Small amines with large effects on plant abiotic stress tolerance. Cells, 9, 2373.
Berg, G. (2009) Plant-microbe interactions promoting plant growth and health: perspectives for controlled use of microorganisms in agriculture. Appl Microbiol Biotechnol, 84, 11-18.
Bharti, N., Pandey, S.S., Barnawal, D., Patel, V.K. and Kalra, A. (2016) Plant growth promoting rhizobacteria Dietzia natronolimnaea modulates the expression of stress responsive genes providing protection of wheat from salinity stress. Sci Rep, 6, 34768.
Calvo, P., Nelson, L. and Kloepper, J.W. (2014) Agricultural uses of plant biostimulants. Plant Soil, 383, 3-41.
Chen, L., Liu, Y, Wu, G., Veronican Njeri, K., Shen, Q., Zhang, N. and Zhang, R. (2016) Induced maize salt tolerance by rhizosphere inoculation of Bacillus amyloliquefaciens SQR9. Physiol Plant, 158, 34-44.
Davey, M.W., Stals, E., Panis, B., Keulemans, J. and Swennen, R.L. (2005) High-throughput determination of malondialdehyde in plant tissues. Anal Biochem, 347, 201-207.
Fernandez, O., Theocharis, A., Bordiec, S., Feil, R., Jacquens, L., Clément, C., Fontaine, F. and Barka, E.A. (2012) Burkholderia phytofirmans PsJN acclimates grapevine to cold by modulating carbohydrate metabolism. MPMI, 25, 496-504.
Habib, S.H., Kausar, H. and Saud, H.M. (2016) Plant growth-promoting Rhizobacteria enhance salinity stress tolerance in Okra through ROS-scavenging enzymes. BioMed Res Int, 2016, e6284547.
Harvey, D.J. (1982) Picolinyl esters as derivatives for the structural determination of long chain branched and unsaturated fatty acids. Biomed Mass Spectrom, 9, 33-38.
Kakar, K.U., Ren, X. -I., Nawaz, Z., Cui, Z.-Q., Li, B., Xie, G.-L., Hassan, M.A., Ali, E. and Sun, G.-C. (2016) A consortium of rhizobacterial strains and biochemical growth elicitors improve cold and drought stress tolerance in rice (Oryza sativa L.). Plant Biol, 18, 471-483.
Kang, S.-M., Khan, A.L., Waqas, M., You, Y.-H., Hamayun, M., Joo, G.-J., Shahzad, R., Choi, K.-S. and Lee, I.-J. (2015) Gibberellin-producing Serratia nematodiphila PEJ1011 ameliorates low temperature stress in Capsicum annuum L. Eur J Soil Biol, 68, 85-93.
Kim, O.-S., Cho, Y.-J., Lee, K., et al. (2012) Introducing EzTaxon-e: a prokaryotic 16S rRNA gene sequence database with phylotypes that represent uncultured species. Int J Syst Evol, 62, 716-721.
Kumar, S., Stecher, G. and Tamura, K. (2016) MEGA7: Molecular Evolutionary Genetics Analysis Version 7.0 for Bigger Datasets. Mol Biol Evol, 33, 1870-1874.
Kumawat, K.C., Sharma, B., Nagpal, S., Kumar, A., Tiwari, S. and Nair, R.M. (2023) Plant growth-promoting rhizobacteria: Salt stress alleviators to improve crop productivity for sustainable agriculture development. Front Plant Sci, 13, 1101862.
Li, J., Wang, J., Liu, H., Macdonald, C.A. and Singh, B.K. (2022) Application of microbial inoculants significantly enhances crop productivity: A meta-analysis of studies from 2010 to 2020. J SustAgr Environ, 1, 216-225.
Marcé, M., Brown, D.S., Capell, T., Figueras, X. and Tiburcio, A.F. (1995) Rapid high-performance liquid chromatographic method for the quantitation of polyamines as their dansyl derivatives: application to plant and animal tissues. J Chromatogr B: Biomed Appl, 666, 329-335.
Mitra, D., Diaz Rodriguez, A.M., Parra Cota, F.I., et al. (2021) Amelioration of thermal stress in crops by plant growth-promoting rhizobacteria. Physiol Mol Plant Pathol, 115, 101679.
Moss, C.W. and Lambert-Fair, M.A. (1989) Location of double bonds in monounsaturated fatty acids of Campylobacter cryaerophila with dimethyl disulfide derivatives and combined gas chromatography-mass spectrometry. J Clin Microbiol, 27, 1467-1470.
Patani, A., Prajapati, D., Ali, D., Kalasariya, H., Yadav, V.K., Tank, J., Bagatharia, S., Joshi, M. and Patel, A. (2023) Evaluation of the growth-inducing efficacy of various Bacillus species on the salt-stressed tomato (Lycopersicon esculentum Mill.). Front Plant Sci, 14, 1168155.
Sambrook, J. and Russell, D.W. (2001) Mutagenesis. In Molecular Cloning: A Laboratory Manual. CSHL Press, p. 13.1-13.105.
Sasser, M. (1990) Identification of bacteria by gas chromatography of cellular fatty acids. MIDI, Technical Note 101.
Spitzer, V. (1996) Structure analysis of fatty acids by gas chromatography — Low resolution electron impact mass spectrometry of their 4,4-dimethyloxazoline derivatives - A review. Prog Lipid Res, 35, 387-408.
Su, F., Jacquard, C., Villaume, S., Michel, J., Rabenoelina, F., Clément, C., Barka, E.A., Dhondt-Cordelier, S. and Vaillant-Gaveau, N. (2015) Burkholderia phytofirmans PsJN reduces impact of freezing temperatures on photosynthesis in Arabidopsis thaliana. Front Plant Sci, 6, 810.
Subramanian, S. and Smith, D.L. (2015) Bacteriocins from the rhizosphere microbiome - from an agriculture perspective. Front Plant Sci, 6, 909.
Tindall, B.J., Sikorski, J., Smibert, R.A. and Krieg, N.R. (2007) Phenotypic characterization and the principles of comparative systematics. In Methods for General and Molecular Microbiology. John Wiley & Sons, Ltd, pp. 330-393.
Vieira, S., Huber, K.J., Neumann-Schaal, M., Geppert, A., Luckner, M., Wanner, G. and Overmann, J. (2021) Usitatibacter rugosus gen. nov., sp. nov. and Usitatibacter palustris sp. nov., novel members of Usitatibacteraceae fam. nov. within the order Nitrosomonadales isolated from soil. Int J Sys Evol Microb, 71, 004631.
Wang, C., Wang, Cui, Gao, Y.-L., Wang, Y.-P. and Guo, J.-H. (2016) A consortium of three plant growth-promoting Rhizobacterium strains acclimates Lycopersicon esculentum and confers a better tolerance to chilling stress. J Plant Growth Regul, 35, 54-64.
Wang, Q., Dodd, I.C., Belimov, A.A., Jiang, F., Wang, Q., Dodd, I.C., Belimov, A.A. and Jiang, F. (2016) Rhizosphere bacteria containing 1-aminocyclopropane-1-carboxylate deaminase increase growth and photosynthesis of pea plants under salt stress by limiting Na+ accumulation. Functional Plant Biol., 43, 161-172.
Yu, Q.T., Liu, B.N., Zhang, J.Y. and Huang, Z.H. (1988) Location of methyl branchings in fatty acids: Fatty acids in uropygial secretion of Shanghai Duck by GC-MS of 4,4-dimethyloxazoline derivatives. Lipids, 23, 804-810.

### Clauses

For reasons of completeness, various aspects of the invention are set out in the following numbered clauses:
**Clause 1.** A *Pararhizobium* strain selected from:
   (a) a strain deposited in the Spanish Type Culture Collection (CECT) with the accession number CECT30825,
   (b) a strain deposited in the Spanish Type Culture Collection (CECT) with the accession number CECT30826, or
   (c) a mutant thereof, wherein the mutant: (i) is obtained from CECT30825 or CECT30826 strain, and (ii) retains the properties of inducing: (ii.1) tolerance to temperatures below 0°C and/or to saline stress to plants.; and (ii.2) plant growth.
**Clause 2.** A bacterial culture comprising the strain or the mutant thereof according to clause 1.
**Clause 3.** The bacterial culture of clause 2, which is an inoculation product.
**Clause 4.** A supernatant derived from the strain as defined in clause 1, said supernatant being obtainable by a process comprising: (i) inoculating the strain in a suitable culture medium; (ii) subjecting the inoculated culture medium to suitable growth conditions; (iii) separating the cells from the culture medium of step (ii); (iv) collecting the supernatant; and (v) optionally subjecting the supernatant to a concentration step.
**Clause 5.** A process for obtaining a viable cell suspension derived from the strain as defined in clause 1, the process comprising: (i) inoculating the strain in a culture medium, (ii) subjecting the inoculated culture medium of the step (i) to conditions suitable for growth of the strain, and (iii) optionally subjecting the medium resulting from step (ii) to a concentration step.
**Clause 6.** A method to obtain a mutant of the strain as defined in clause 1, comprising the step of subjecting the strain CECT30825 or CECT30826 to a DNA recombinant technique, preferably mutagenesis.
**Clause 7**. A composition comprising the strain according to clause 1, the bacterial culture according to any one of the clauses 2-3 or the supernatant according to clause 4, and one or more agriculturally acceptable compounds.
**Clause 8.** The composition according to clause 7 wherein the strain is present at a concentration from 10⁵CFU/ml to 10¹²CFU/ml, particularly from 10⁷ to 10¹⁰ CFU/mL, particularly 10⁸ CFU/mL.
**Clause 9.** The composition according to any one of clauses 7-8, wherein the agriculturally acceptable compound is selected from the group consisting of: plant strengtheners, nutrients, wetting agents, compounds that improve adherence, buffering compounds, stabilizers, antioxidants, osmotic protectors and sunscreens.
**Clause 10.** The composition according to any one of clauses 7-9, which comprises at least one additional growth promoting agent.
**Clause 11.** The composition according to any one of the clauses 7-10, which comprises the strain CECT30285.
**Clause 12.** The composition according to clause 11, which further comprises the strain CECT30286.
**Clause 13.** A kit comprising an effective amount of the strain as defined in clause 1, the bacterial culture according to any of the clauses 2-3, the supernatant according to clause 4, or the composition according to any one of clauses 7 to 12.
**Clause 14.** Use of the *Pararhizobium* strain as defined in clause 1, the bacterial culture as defined in any of the clauses 2-3, the supernatant as defined in clause 4, or the composition as defined in any of the clauses 7-12, as a growth plant promoter.
**Clause 15.** Use of the *Pararhizobium* strain as defined in clause 1, the bacterial culture as defined in any of the clauses 2-3, the supernatant as defined in clause 4, or the composition as defined in any of the clauses 7-12, for increasing abiotic stress tolerance, biomass and/or yield of a plant.
**Clause 16.** A method for promoting the growth in a plant, the method comprising applying to the plant the strain as defined in clause 1, the bacterial culture as defined in any of the clauses 2-3, the supernatant as defined in clause 4, or the composition as defined in any of the clauses 7-12.
**Clause 17.** A method for increasing abiotic stress tolerance, biomass and/or yield of a plant, the method comprising applying to the plant the strain as defined in clause 1 or 9, the bacterial culture as defined in any of the clauses 2-3, the supernatant as defined in clause 4, or the composition as defined in any of the clauses 7-12.
**Clause 18.** The method of any one of the clauses 16-17, wherein the application is performed by spraying on aerial parts of the plant or by soil-inoculation.
**Clause 19.** The use of clause 15 or the method of clause 17, wherein the abiotic stress is selected from the group consisting of: chilling temperature; freezing temperature; and saline stress.

## Claims

1. A *Pararhizobium* strain selected from:
(a) a strain deposited in the Spanish Type Culture Collection (CECT) with the accession number CECT30825,
(b) a strain deposited in the Spanish Type Culture Collection (CECT) with the accession number CECT30826, or
(c) a mutant thereof, wherein the mutant: (i) is obtained from CECT30825 or CECT30826 strain, and (ii) retains one or more of the following properties: (ii.1) induction of tolerance to chilling temperature; (ii.2) induction of tolerance to freezing temperature; (ii.3) induction of plant growth; and (ii.4) increased yield.

2. A bacterial culture comprising the strain or the mutant thereof according to claim 1.

3. The bacterial culture of claim 2, which is an inoculation product.

4. A supernatant derived from the strain as defined in claim 1, said supernatant being obtainable by a process comprising: (i) inoculating the strain in a suitable culture medium; (ii) subjecting the inoculated culture medium to suitable growth conditions; (iii) separating the cells from the culture medium of step (ii); (iv) collecting the supernatant; and (v) optionally subjecting the supernatant to a concentration step.

5. A process for obtaining a viable cell suspension derived from the strain as defined in claim 1, the process comprising: (i) inoculating the strain in a culture medium, (ii) subjecting the inoculated culture medium of the step (i) to conditions suitable for growth of the strain, and (iii) optionally subjecting the medium resulting from step (ii) to a concentration step.

6. A method to obtain a mutant of the strain as defined in claim 1, comprising the step of subjecting the strain CECT30825 or CECT30826 to a DNA recombinant technique, preferably mutagenesis.

7. A composition comprising the strain according to claim 1, the bacterial culture according to any one of the claims 2-3 or the supernatant according to claim 4, and one or more agriculturally acceptable compounds.

8. The composition according to claim 7 wherein the strain is present at a concentration from 10⁵CFU/ml to 10¹²CFU/ml, particularly from 10⁷ to 10¹⁰ CFU/mL, particularly 10⁸ CFU/mL.

9. The composition according to any one of claims 7-8, which comprises at least one additional growth promoting agent; particularly the composition comprises the strain CECT30285; particularly the composition comprises the strain CECT30825 and CECT30286.

10. A kit comprising an effective amount of the strain as defined in claim 1, the bacterial culture according to any of the claims 2-3, the supernatant according to claim 4, or the composition according to any one of claims 7 to 9.

11. Use of the *Pararhizobium* strain as defined in claim 1, the bacterial culture as defined in any of the claims 2-3, the supernatant as defined in claim 4, or the composition as defined in any of the claims 7-12, as a growth plant promoter.

12. Use of the *Pararhizobium* strain as defined in claim 1, the bacterial culture as defined in any of the claims 2-3, the supernatant as defined in claim 4, or the composition as defined in any of the claims 7-9, for increasing abiotic stress tolerance, biomass and/or yield of a plant.

13. A method for promoting the growth in a plant, the method comprising applying to the plant the strain as defined in claim 1, the bacterial culture as defined in any of the claims 2-3, the supernatant as defined in claim 4, or the composition as defined in any of the claims 7-9.

14. A method for increasing abiotic stress tolerance, biomass and/or yield of a plant, the method comprising applying to the plant the strain as defined in claim 1 or 9, the bacterial culture as defined in any of the claims 2-3, the supernatant as defined in claim 4, or the composition as defined in any of the claims 7-9.

15. The use of claim 12 or the method of claim 14, wherein the abiotic stress is selected from the group consisting of:
- chilling temperature;
- freezing temperature; and
- saline stress.
